# EUROPÄISCHE PATENTSCHRIFT

(11) **EP 2 939 699 B1**
(45) Veröffentlichungstag und Bekanntmachung des Hinweises auf die Patenterteilung: **26.12.2018**
(21) Anmeldenummer: 15160726.4
(22) Anmeldetag: 25.03.2015
(51) Int. Cl.: A61M 3/02, B05B 17/06, F04B 7/04, F04B 9/12, B25D 17/06, B25D 9/08

(54) **DRUCKGASMOTOR ZUM BETREIBEN EINES LAVAGE-SYSTEMS**
COMPRESSED GAS MOTOR FOR OPERATING A LAVAGE SYSTEM
MOTEUR À GAZ COMPRIMÉ DESTINÉ AU FONCTIONNEMENT D'UN SYSTÈME DE LAVAGE

(30) Priorität: 29.04.2014 DE 102014208064
(43) Veröffentlichungstag der Anmeldung: 04.11.2015
(73) Patentinhaber: Heraeus Medical GmbH, 61273 Wehrheim (DE)
(72) Erfinder: Vogt, Sebastian, 99092 Erfurt (DE)
(74) Vertreter: Heraeus IP

(56) Entgegenhaltungen:
- DE-A1- 3 239 174
- DE-A1-102011 018 708
- DE-B3-102010 046 057
- JP-A- H0 355 175
- US-A- 3 937 055
- US-A- 5 542 918

## Beschreibung

Die Erfindung betrifft ein Lavage-System mit einem einen Kolben und ein Rückstellelement aufweisenden Druckgasmotor.

Die Erfindung betrifft ferner ein Verfahren zum Erzeugen eines Sprühstoßes.

Gegenstand der Erfindung ist somit ein Lavage-System mit einem vereinfachten Druckgasmotor, der zum Teil aus kostengünstigen Kunststoffen gefertigt werden kann und als Pump-Antrieb in den oben beschriebenen medizinischen Spülvorrichtungen angewendet wird. Der Druckgasmotor selbst ist auch für andere Zwecke einsetzbar.

In der Chirurgie werden medizinische Spülsysteme in breitem Umfang eingesetzt, um Gewebeareale zu reinigen. Diese Spülsysteme werden als Lavage-Systeme bezeichnet. Mit den Lavage-Systemen werden mit den Spülflüssigkeiten Sprühstrahlen erzeugt, die auf die zu reinigenden Gewebeareale auftreffen und eine mechanische Reinigungswirkung auf diese Gewebeareale ausüben. Insbesondere bei der Implantation von Gelenkendoprothesen und bei septischen Revisionen sind Lavage-Systeme von wesentlicher Bedeutung (R. M. Sherman et al.: The role of lavage in preventing hemodynamic and blood-gas changes during cemented arthroplasty. J. Bone Joint. Surg. 1983; 65-A: 500-506; S. J. Breusch et al.: Zementierte Hüftendoprothetik: Verminderung des Fettembolierisikos in der zementierten Hüftendoprothetik mittels gepulster Druckspülung. Orthopädie 2000; 29: 578-586; S. J. Breusch et al.: Lavage technique in THA: Jetlavage Produces Better Cement Penetration Than Syringe-Lavage in the Proximal Femur. J. Arthroplasty. 200; 15(7): 921-927; R. J. Byrick et al.: High-volume, high pressure pulsatile lavage during cemented arthroplasty. J. Bone Joint Surg. 1989; 81-A: 1331-1336; J. Christie et al.: Medullary lavage reduces embolic phenomena and cardiopulmonary changes during cemented hemiarthorplasty. J. Bone Joint Surg. 1995; 77-B: 456-459).

Gepulste Lavage-Systeme sind seit langem bekannt, beispielsweise aus der US 4,583,531 A, der US 4,278,078 A und der US 5,542,918 A. Die gegenwärtig im Markt befindlichen Lavage-Systeme werden durch Elektromotoren (zum Beispiel Inter-Pulse® Jet Lavage der Stryker GmbH & Co. KG) oder durch Druckluft (zum Beispiel PALAVAGE® der Heraeus Medical GmbH) angetrieben. Bewährt haben sich auch handgehaltene, elektrisch angetriebene Lavage-Systeme. Es muss jedoch immer ein großer Batterieblock oder Akkumulatorenblock mitgeführt werden, der naturgemäß nur eine begrenzte Ladungskapazität hat. Batterie- und Akkumulatorenblöcke sind im Hinblick auf ihre Umweltfreundlichkeit kritisch zu sehen. Druckgasgetriebene Lavage-Systeme haben den Vorteil, dass Druckluft im Operationssaal meist unlimitiert zur Verfügung steht und damit beliebig lange Spülflüssigkeit versprüht werden kann, ohne dass die Energiezufuhr begrenzt ist.

Bei der Verwendung von Druckluft-getriebenen Lavage-Systemen muss jedoch eine Doppelschlauchsystem verwendet werden, bei dem in einem Schlauch die unsterile Druckluft zugeführt wird und einen zweiten Schlauch mit dem die nach dem Antrieb des Druckluftmotors zumindest teilentspannte unsterile Luft abgeführt wird. Bei den mit Druckluft oder einem anderen komprimierten Gas getriebenen Systemen wird jedoch üblicherweise ein Druckgasmotor zum Antrieb eingesetzt. Bei den meisten Druckgasmotoren für Lavage-Systeme handelt es sich um Lamellen-Druckgasmotoren. Der Druckgasmotor erzeugt eine Drehbewegung, die dann in eine oszillierende, lineare Bewegung umgesetzt wird. Die oszillierende lineare Bewegung wird genutzt, um jeweils kleinen Volumina des Spülmediums einen Impuls zu verleihen. Dabei wird üblicherweise mindestens eine Membran zwischen dem Antrieb und dem Zulauf der Spülflüssigkeit angeordnet, um die Impulse auf die Spülflüssigkeit übertragen zu können. Es entstehen dadurch Sprühstöße mit hohen Pulszahlen von 2000 bis 3000 Impulsen pro Minute. Das bedeutet, dass der Druckgasmotor sehr präzise gefertigt sein muss, um entsprechend hohe Drehzahlen zu tolerieren. Weiterhin muss eine entsprechend stabile Lagerung vorhanden sein. Aus diesen Gründen ist der Druckgasmotor bei üblichen Druckgas-getriebenen Lavage-Systemen das kostenintensivste Bauteil. Es wird deshalb im Allgemeinen der Druckgasmotor in einem Handgriff aus Metall oder anderen langzeitstabilen Materialien angeordnet, so dass dieses Bauteil mehrfach nach entsprechender Aufbereitung und Sterilisation genutzt werden kann. Druckgasmotoren nutzen den Druckunterschied zwischen dem zum Antrieb eingesetzten komprimierten Gas und der Druck der umgebenden Atmosphäre. Theoretisch können diese auch mit einem Unterdruck am Gasauslass betrieben werden, da es nur auf die Druckdifferenz ankommt.

Aus der DE 10 2010 046 057 B3 ist ein Druckgasmotor bekannt, bei dem ein Kolbensystem eine periodische Bewegung erzeugt, die auch zum Pumpen von Flüssigkeiten verwendet werden kann.

Ein Vorschlag zur Erzeugung von gepulsten Flüssigkeitsstrahlen wird in der DE 10 2011 018 708 A1 beschreiben. Dabei wirkt ein Kolbenvibrator periodisch auf eine Membran ein. Die Membran bildet mit einem Hohlraum eine Pumpe.

Nachteilig bei allen Pumpen mit Membran ist jedoch, dass es sehr schwierig ist, eine geeignete Membran mit hoher Rückstellkraft herzustellen, die den hohen Pulszahlen eines Kolbenvibrators beziehungsweise Vibrationskörpers folgen kann. Dadurch sind hohe Pulszahlen nur aufwendig realisierbar. Weiterhin sind solche Systems auf Grund der axialen Verformbarkeit der Membran anfällig, da der Vibrationskörper sich axial zu weit in Richtung Membran bewegen und dadurch stehen bleiben kann. Solche Systeme sind nicht ausreichend robust.

Andere Druckgasmotoren können blockieren, indem die Kolben im Hubraum verkanten und dadurch blockieren. Zudem kann es passieren, dass die Kolben eine Stellung einnehmen, bei der der Druckgasmotor nicht ohne weiteres wieder von alleine anläuft.

Druckgasmotoren sind nicht nur für Lavage-Systeme geeignet, sondern können auch überall dort eingesetzt werden, wo ein Druckgas zur Verfügung steht und ein kostengünstiger Antrieb vorteilhaft ist. Solche Voraussetzungen sind beispielsweise bei Rüttelanlagen gegeben, bei denen ein Schüttgut oder Pulver transportiert, abgefüllt beziehungsweise portioniert werden muss. Ebenso können solche Druckgasmotoren als Pumpen zur Bereitstellung von Schmiermitteln vorteilhaft eingesetzt werden.

Die Aufgabe der Erfindung besteht also darin, die Nachteile des Stands der Technik zu überwinden. Insbesondere soll ein kostengünstiger und zuverlässiger Druckgasmotor gefunden werden, der für die genannten Zwecke einsetzbar ist. Die Erfindung liegt damit die Aufgabe zugrunde, eine robuste, maximal vereinfachte medizinische Spülvorrichtung zu entwickeln, die einen gepulsten Flüssigkeitsstrahl erzeugen kann, wobei die Pulsrate insbesondere größer als 1000 Pulse pro Minute sein soll. Die Vorrichtung soll wenn möglich weitegehend aus kostengünstigen Materialien gefertigt werden können und soll durch Druckgas angetrieben werden können. Die zu entwickelnde medizinische Spülvorrichtung kann für den einmaligen Gebrauch bestimmt sein. Die Spülvorrichtung soll sowohl für den Betrieb mit Druckluft aus stationären Druckluftversorgungssystemen als auch für Betrieb mit Druckgasen aus mobilen Druckgasflaschen oder Gaspatronen geeignet sein.

Eine Aufgabe der Erfindung ist dabei darin zu sehen, einen maximal vereinfachten Kolben-Druckgasmotor zu entwickeln, der eine periodische, lineare Kolbenbewegung erzeugen kann. Der Druckgasmotor soll mit dem Druckgas von im Krankenhaus üblichen stationären Druckgasanlagen betrieben werden können. Es soll ferner ein Verfahren zur Erzeugung einer linearen, periodischen Bewegung entwickelt werden, wobei der zu entwickelnde Druckgasmotor verwendet werden soll oder zumindest verwendbar sein soll. Dabei ist es wichtig, dass der Druckgasmotor ohne komplexe, kostenintensive Ventilsysteme auskommt und so vereinfacht wird, dass möglichst viele Bauteile des Druckgasmotors kostengünstig durch Kunststoffspritzgießen oder durch Drehen von gut bearbeitbaren Metallkörpern hergestellt werden können. Der Druckgasmotor soll für den Antrieb einer medizinischen Spülvorrichtung verwendbar sein. Es müssen Ventilsysteme vermieden werden, die ein großes Volumen einnehmen und die separat vom Druckgasmotor positioniert werden müssen. In dem Druckgasmotor sollen daher die erforderlichen Ventilfunktionen möglichst platzsparend integriert sein, um eine Verwendung des Druckgasmotors als Antrieb in Handstücken von Lavage-Systemen zu ermöglichen. Die zeitliche Steuerung der Ventilfunktionen sollte idealerweise so sein, dass bei jedem Punkt der Kolbenbewegung ein "toter Punkt" vermieden wird. Weiterhin soll mit dem Druckgasmotor eine vereinfachte Pumpe entwickelt werden, die in handgehaltenen Lavage-Systemen untergebracht werden kann. Eine mit dem Druckgasmotor angetriebene Pumpe soll so vereinfacht sein, das diese derart kostengünstig hergestellt werden kann, dass auch eine Verwendung in Lavage-Systemen möglich ist, die zur nur einmaligen Verwendung bestimmt sind.

Die Aufgaben der Erfindung werden gelöst durch einen Druckgasmotor aufweisend einen Vibrationskörper, der mit einem durch den Druckgasmotor geleiteten Druckgas in Schwingung versetzbar ist, und einen Kolben, der mit einem Rückstellelement federnd gelagert ist, wobei der Vibrationskörper und der Kolben derart positioniert und gelagert sind, dass der Vibrationskörper bei der Schwingung wiederholt auf den Kolben trifft und diesen gegen das Rückstellelement auslenkt, wobei der Kolben von dem Rückstellelement in eine Position überführbar ist, in der der Vibrationskörper bei seiner Schwingung den Kolben trifft, so dass der Vibrationskörper bei der Schwingung wiederholt auf den Kolben trifft, und wobei die Bewegung des Kolbens als Antrieb des Druckgasmotors nutzbar ist.

Unter einem Druckgasmotor ist im Rahmen der vorliegenden Erfindung ein Motor zu verstehen, der mit Hilfe eines Druckgefälles zwischen einem ersten Gas (dem Druckgas) und einem zweiten Gas (beispielsweise der Umgebung) mit einem niedrigeren Druck Arbeit leisten kann. Dabei wird als Arbeitsmedium bevorzugt ein komprimiertes Druckgas verwendet und an die Umgebung des Druckgasmotors abgeleitet. Durch die Strömung des Druckgases durch den Druckgasmotor werden der Vibrationskörper und damit der Kolben in eine periodische Bewegung versetzt.

Bei erfindungsgemäßen Druckgasmotoren kann vorgesehen sein, dass das Rückstellelement während des Betriebs des Druckgasmotors zumindest zeitweise eine in Richtung des Vibrationskörpers wirkende Kraft auf den Kolben ausübt.

Bevorzugt bewirkt das Rückstellelement bei jeder Stellung des Kolbens außer der Ausgangsposition oder der Ruheposition des Kolbens eine Kraft in Richtung des Vibrationskörpers.

Hierdurch wird sichergestellt, dass der Vibrationskörper während der Schwingung wiederholt auf den Kolben treffen kann und der Kolben hierdurch zu einer Schwingung angeregt wird, die als Antrieb für den Druckgasmotor nutzbar ist.

Mit der Erfindung wird auch vorgeschlagen, dass der Druckgasmotor einen Innenraum aufweist, wobei der Vibrationskörper und der Kolben in dem Innenraum linear beweglich angeordnet sind und der Innenraum durch eine Vorderseite, eine geschlossene Rückseite und eine umlaufende Seitenwand begrenzt ist, wobei der Kolben zwischen der Vorderseite und dem Vibrationskörper angeordnet ist.

Die Querschnittsflächen der beiden Bereiche des Innenraums sind senkrecht zur linearen Bewegungsrichtung des Vibrationskörpers und bevorzugt auch senkrecht zur linearen Bewegungsrichtung des Kolbens orientiert.

Gemäß einer bevorzugten Ausgestaltung der Erfindung kann vorgesehen sein, dass der vordere Bereich und der hintere Bereich des Innenraums eine zylindrische Geometrie mit kreisförmiger Grundfläche aufweisen. Die beiden Bereiche des Innenraums sind dabei bevorzugt geometrisch über ihre Grundflächen miteinander verbunden. Bevorzugt liegen die Zylinderachsen der zylindersymmetrischen Bereiche des Innenraums auf der gleichen Achse. Der Kolben weist dazu eine Zylindersymmetrie mit kreisförmiger Grundfläche auf, wobei der Radius des Kolbens so groß wie oder etwas kleiner als der Durchmesser des vorderen Bereichs des Innenraums gewählt ist, beziehungsweise der Radius des Kolbens zum Innenradius des Innenraums passend gewählt ist. Der Vibrationskörper ist erfindungsgemäß bevorzugt zusammengesetzt aus zwei zylindrischen Körpern mit kreisförmiger Grundfläche und unterschiedlichen Radien. Die Radien sind dabei so gewählt, dass die zwei zylindrischen Teile (Körper) des Vibrationskörpers in den vorderen Bereich und den hinteren Bereich des Innenraums passen. Der Kolben und der Vibrationskörper sollen in dem Innenraum beweglich sein, den Innenraum aber gleichzeitig über deren Außenumfänge abdichten.

Ein solcher Innenraum schließt den Druckgasmotor nach Außen weitgehend ab. Dies ist für die Sicherheit des Betriebs vorteilhaft, bietet aber auch eine ganze Reihe von Möglichkeiten bei Verwendung des Druckgases als Antrieb. Beispielsweise kann eine Gasfederung zur Erzeugung der periodischen Bewegung des Vibrationskörpers genutzt werden und der Vibrationskörper kann nach Art eines Kolbens durch das sich im Innenraum ausdehnende Druckgas genutzt werden.

Bei erfindungsgemäßen Druckgasmotoren kann vorgesehen sein, dass der Innenraum einen vorderen Bereich mit einer kleinen Querschnittsfläche und einen hinteren Bereich mit einer großen Querschnittsfläche aufweist, die größer als die kleine Querschnittsfläche ist, wobei der Kolben in dem vorderen Bereich des Innenraums zwischen der Vorderseite und dem Vibrationskörper linear beweglich angeordnet ist.

Hierdurch kann eine kraftvolle Bewegung des Vibrationskörpers erreicht werden, so dass der Kolben mit großem Impuls angetrieben werden kann.

Dabei kann wiederum vorgesehen sein, dass der Vibrationskörper einen vorderen Teil mit einer kleinen Querschnitt passend zur kleinen Querschnittsfläche des vorderen Bereichs des Innenraums aufweist, so dass der vordere Teil des Vibrationskörpers den vorderen Bereich des Innenraums an der der Rückseite zugewandten Seite im Betrieb des Druckgasmotors zumindest zeitweise abschließt, und der Vibrationskörper einen hinteren Teil mit einem großen Querschnitt passend zur großen Querschnittsfläche des hinteren Bereichs des Innenraums aufweist, so dass der hintere Teil des Vibrationskörpers den hinteren Bereich des Innenraums in zwei Teile trennt.

Hierdurch wird eine Gasfederung des Vibrationskörpers erreicht, die in eine zum Kolben entgegengesetzte Richtung wirkt. Wenn der vordere Teil des Vibrationskörpers den vorderen Bereich des Innenraums an der der Rückseite zugewandten Seite im Betrieb des Druckgasmotors nur zeitweise abschließt, also zeitweise offen ist, oder auf andere Art und Weise während der Bewegung des Vibrationskörpers frisches Gas in diesen Zwischenraum eingespeist wird, kann sichergestellt werden, dass stets ein ausreichender Gasdruck zur Federung des Vibrationskörpers vorhanden ist.

Ferner wird mit der Erfindung vorgeschlagen, dass in der Seitenwand im hinteren Bereich des Innenraums eine Gaseinlassöffnung zum Einspeisen eines Gases in den Innenraum vorgesehen ist und in der Seitenwand im vorderen Bereich des Innenraums eine Gasauslassöffnung zum Abführen des Gases aus dem Innenraum vorgesehen ist.

Es kann erfindungsgemäß vorgesehen sein, dass die Gaseinlassöffnung an eine Druckgasquelle anschließbar ist. Der Druckgasmotor kann dann beispielsweise mit einem Druckgas aus einem Kompressor oder einer Druckgasflasche oder einer Flüssiggaspatrone, wie beispielsweise einer CO₂-Patrone, betrieben werden. Die Abluft, die den Druckluftmotor durch die Gasauslassöffnung verlässt, kann dann an die Umgebung abgegeben werden.

Hierdurch wird sichergestellt, dass das Druckgas am Vibrationskörper vorbei oder durch diesen hindurch geleitet wird und so zum Antrieb des Vibrationskörpers genutzt werden kann.

Mit einer bevorzugten Ausgestaltung der vorliegenden Erfindung wird auch vorgeschlagen, dass der Innenraum zwischen dem Kolben und der Rückseite des Innenraums bis auf die Gaseinlassöffnung und die Gasauslassöffnung geschlossen ist.

Hierdurch wird verhindert, dass der Druckgasmotor Leistung durch entweichende Druckluft einbüßt.

Mit einer Weiterbildung der vorliegenden Erfindung wird vorgeschlagen, dass in dem Vibrationskörper wenigstens ein Kanal zur Gasleitung vorgesehen ist, wobei der Kanal oder die Kanäle den hinteren Bereich des hinteren Innenraums zwischen dem Vibrationskörper und der Rückseite des Innenraums abhängig von der Position des Vibrationskörpers in dem Innenraum mit der Gaseinlassöffnung oder mit der Gasauslassöffnung verbindet oder verbinden.

Hierdurch wird eine kompakte und für Fehler unanfällige Bauweise des Druckgasmotors erreicht. Der Kanal oder die Kanäle sind dazu gasdurchlässig gestaltet.

Eine besonders bevorzugte Ausführung der vorliegenden Erfindung kann vorsehen, dass der Vibrationskörper in einer ersten Position den hinteren Bereich des Innenraums zwischen dem Vibrationskörper und der Rückseite des Innenraums durch den wenigstens einen Kanal mit der Gaseinlassöffnung verbindet und von der Gasauslassöffnung trennt und in einer zweiten Position den hinteren Bereich des Innenraums zwischen dem Vibrationskörper und der Rückseite des Innenraums durch den wenigstens einen Kanal mit der Gasauslassöffnung verbindet und von der Gaseinlassöffnung trennt.

Mit diesem Aufbau wird sichergestellt, dass es zu keiner Kurzschlussposition kommen kann, in der die Gaseinlassöffnung mit der Gasauslassöffnung über den wenigstens einen Kanal verbunden ist und der Druckgasmotor zu zum Stillstand kommt. Zudem wäre er in einer solchen Position des Vibrationskörpers nicht mehr zu starten.

Dabei kann vorgesehen sein, dass der Vibrationskörper in einer dritten Position zwischen der ersten Position und der zweiten Position sowohl die Gaseinlassöffnung als auch die Gasauslassöffnung abdeckt.

Auch diese Maßnahme dient dazu, dass der Druckgasmotor zuverlässig und ohne stehenzubleiben läuft.

Zur Realisierung der Erfindung wird auch vorgeschlagen, dass der zumindest eine gasdurchlässige Kanal sich von der der Rückseite zugewandten Seite des Vibrationskörpers bis zur seitlichen Mantelfläche des hinteren Teils des Vibrationskörpers erstreckt und der gleiche Kanal oder ein anderer der gasdurchlässigen Kanäle sich von der der Rückseite zugewandten Seite des Vibrationskörpers bis zur seitlichen Mantelfläche des vorderen Teils des Vibrationskörpers erstreckt. Bevorzugt weist der Vibrationskörper nur einen als T-Stück ausgebildeten gasdurchlässigen Kanal auf, der sich von der der Rückseite zugewandten Seite des Vibrationskörpers bis zur seitlichen Mantelfläche des hinteren Teils des Vibrationskörpers und zur seitlichen Mantelfläche des vorderen Teils des Vibrationskörpers erstreckt.

Hierdurch wird ein besonders einfacher und leicht zu realisierender Aufbau des Druckgasmotors erreicht.

Mit einer Weiterbildung der Erfindung kann auch vorgesehen sein, dass im vorderen Bereich des Innenraums in der Seitenwand des Innenraums eine Flüssigkeitseinlassöffnung vorgesehen ist und in der Vorderseite des Innenraums eine Flüssigkeitsauslassöffnung vorgesehen ist, wobei bevorzugt an zumindest einer der Flüssigkeitsöffnungen, besonders bevorzugt an beiden Flüssigkeitsöffnungen, ein Ventil, insbesondere ein Lippenventil angeordnet ist.

Es kann dabei erfindungsgemäß vorgesehen sein, dass die Flüssigkeitseinlassöffnung mit einer Flüssigkeitszuführung und/oder einer Flüssigkeitsquelle verbunden ist. Bevorzugt ist die Flüssigkeit eine medizinische Spülflüssigkeit.

Bei diesen Ausführungsformen kann auch vorgesehen sein, dass an der Flüssigkeitseinlassöffnung ein Ventil angeordnet ist, das öffnet, wenn im vorderen Bereich des Innenraums ein geringer Druck durch eine Bewegung des Kolbens von der Vorderseite weg entsteht, und an der Flüssigkeitsausstoßöffnung ein Ventil angeordnet ist, das öffnet, wenn im vorderen Bereich des Innenraums ein hoher Druck durch eine Bewegung des Kolbens auf die Vorderseite zu entsteht.

Durch die genannten Maßnahmen kann der Druckgasmotor unmittelbar und auf einfache Art und Weise als Pumpe und als medizinisches Spülsystem, insbesondere als Lavage-System verwendet werden.

Gemäß einer Weiterentwicklung der vorliegenden Erfindung wird vorgeschlagen, dass die Flüssigkeitseinlassöffnung im Betrieb des Druckgasmotors zumindest zeitweise nicht vom Kolben abgedeckt ist und die Flüssigkeitseinlassöffnung im nicht abgedeckten Zustand zwischen dem Kolben und der Vorderseite des Innenraums angeordnet ist.

Hierdurch wird eine gute Pumpwirkung sichergestellt.

Ferner kann vorgesehen sein, dass an der Flüssigkeitseinlassöffnung ein Rohr oder eine Schlauch mit einem Rückschlagventil angeschlossen ist, das sich bei einem Unterdruck im vorderen Bereich des Innenraums zwischen dem Kolben und der Vorderseite des Innenraums öffnet und eine Flüssigkeitszufuhr in den vorderen Bereich des Innenraums ermöglicht.

Damit kann erreicht werden, dass keine Flüssigkeit aus dem Druckgasmotor in das Flüssigkeitsreservoir gedrückt werden kann. Dies ist vor allem dann wichtig, wenn der Druckgasmotor zum Versprühen von sterilen medizinischen Flüssigkeiten verwendet wird.

Des Weiteren kann vorgesehen sein, dass das Rückstellelement eine elastische Druckfeder ist, die bevorzugt im vorderen Bereich des Innenraums zwischen dem Kolben und der Vorderseite des Innenraums angeordnet ist.

Dieser Aufbau ist besonders einfach und kostengünstig zu realisieren.

Bevorzugte Ausführungsformen können sich dadurch auszeichnen, dass der Innenraum zumindest bereichsweise zylindrisch ist oder im Bereich eines Arbeitsraums des Vibrationskörpers und/oder des Kolbens oder im gesamten Hubraum des Kolbens und des Vibrationskörpers zylindrisch ist.

Durch diesen Aufbau kann der Druckgasmotor besonders einfach und kostengünstig aufgebaut werden. Durch das Vermeiden von Kanten und Ecken kann ein Blockieren des Druckgasmotors vermieden werden. Zudem sind zylindrische Formen besonders einfach und kostengünstig zu fertigen.

Es wird mit der Erfindung vorgeschlagen, dass der Vibrationskörper eine höhere Dichte als der Kolben aufweist.

Es kann für erfindungsgemäße Druckgasmotoren wesentlich sein, dass der Vibrationskörper eine Dichte von mindestens 4 g/cm³ hat, bevorzugt eine Dichte von mindestens 7 g/cm³ hat.

Durch diese erfindungsgemäß besonders wesentliche Ausgestaltung wird erreicht, dass der Vibrationskörper eine ausreichend hohe kinetische Energie erreicht beziehungsweise eine ausreichend hohen Impuls erhält, um den Kolben mit dem notwendigen Impuls beziehungsweise mit der notwendigen kinetischen Energie in Richtung der Vorderseite des Innenraums zu schlagen, beziehungsweise zu beschleunigen. Die Energie beziehungsweise der Impuls müssen dazu ausreichen, die Federkraft der Druckfeder beziehungsweise die elastische Kraft des Rückstellelements so weit zu überwinden, dass diese weitgehend gestaucht beziehungsweise ausgelenkt wird.

Der Vibrationskörper ist dazu bevorzugt aus einem Metall oder einem hochdichten Kunststoff aufgebaut, bevorzugt aus Messing, Stahl oder einem anderen leicht zu bearbeitenden Metall oder einem mit Schwerspat oder mit Wolfram gefüllten Kunststoff. Der restliche Druckgasmotor bis auf das Rückstellelement kann erfindungsgemäß aus einem Kunststoff wie Polyethylen gefertigt sein und beispielsweise mit einem Spitzgussverfahren hergestellt werden.

Ferner kann vorgesehen sein, dass der Kolben mit dem Rückstellelement in eine Position gebracht wird, in der die Vorderseite des vorderen Teils des Vibrationskörpers bei maximaler Auslenkung des Vibrationskörpers in Richtung der Vorderseite des Innenraums auf die der Rückseite des Innenraums zugewandten Seite des Kolbens trifft und diesen in Richtung der Vorderseite des Innenraums beschleunigt.

Dabei kann erfindungsgemäß bevorzugt vorgesehen sein, dass der Vibrationskörper den Kolben um maximal 3 mm, besonders bevorzugt um 1 mm bis 3 mm, in Richtung der Vorderseite des Innenraums verschiebt.

Die Positionen, die der Kolben und der Vibrationskörper im Betrieb des Druckgasmotors einnehmen können, überlappen sich also ein wenig, bevorzugt um maximal 3 mm, besonders bevorzugt um 1 mm bis 3 mm, in der Bewegungsrichtung des Vibrationskörpers. Hierdurch soll sichergestellt werden, dass der Vibrationskörper seinen Impuls auf den Kolben übertragen kann.

Mit einer Weiterbildung der Erfindung wird vorgeschlagen, dass der vordere Teil des Vibrationskörpers den vorderen Bereich des Innenraums vom hinteren Bereich des Innenraums trennt, wenn der vordere Teil des Vibrationskörpers in dem vorderen Bereich des Innenraums angeordnet ist.

Hierdurch wird eine Gasfederung des Vibrationskörpers gegen den der Vorderseite des Innenraums zugewandten Teil des hinteren Innenraums erzeugt, die durch die der Vorderseite des Innenraums zugewandte Grundfläche des hinteren Teils des Vibrationskörpers rückseitig begrenzt wird. Diese Gasfederung treibt den Vibrationskörper in Richtung der Rückseite des Innenraums und damit zurück in seine Ausgangslage.

Bevorzugt kann dabei vorgesehen sein, dass der Vibrationskörper derart geformt und im Innenraum gelagert ist, dass der vordere Teil des Vibrationskörpers in jeder Stellung des Vibrationskörpers in dem vorderen Bereich des Innenraums angeordnet ist. Dadurch bleibt der vordere Bereich des Innenraums zwischen dem Kolben und dem Vibrationskörper immer von der Gasfederung getrennt, also von dem der Vorderseite des Innenraums zugewandten Teil des hinteren Innenraums getrennt, der durch die der Vorderseite des Innenraums zugewandte Grundfläche des hinteren Teils des Vibrationskörpers rückseitig begrenzt wird.

Gemäß einer weiteren Ausgestaltung der Erfindung kann auch vorgesehen sein, dass der Kolben vollumfänglich an der Innenwand des vorderen Bereichs des Innenraums anliegt, bevorzugt vollumfänglich, gasdicht und druckdicht an der Innenwand des vorderen Bereichs des Innenraums anliegt, und/oder der Vibrationskörper vollumfänglich an der Innenwand des hinteren Bereichs des Innenraums anliegt, bevorzugt vollumfänglich, gasdicht und druckdicht an der Innenwand des hinteren Bereichs des Innenraums anliegt.

Hierdurch kann ein Druckgasmotor mit einem hohen Wirkungsgrad erreicht werden, da möglichst die gesamte Druckluft zum Antreiben des Vibrationskörpers und damit zum Leisten der Arbeit verwendet werden kann.

Erfindungsgemäß bevorzugt kommen dabei keine gummielastischen Dichtungsringe zum Einsatz. Der Verzicht auf gummielastische Dichtungen hat den Vorteil, dass in dem Druckgasmotor weniger Reibung entsteht und dadurch der Druckgasmotor wesentlich schneller beziehungsweise mit einer höheren Frequenz laufen kann.

Es kann erfindungsgemäß auch vorgesehen sein, dass der Vibrationskörper vollumfänglich an der Innenwand des vorderen Bereichs des Innenraums anliegt, bevorzugt vollumfänglich, gasdicht und druckdicht an der Innenwand des vorderen Bereichs des Innenraums anliegt. Hierdurch kann der hintere Teil des Innenraums von dem vorderen Teil des Innenraums getrennt werden.

Es kann erfindungsgemäß vorgesehen sein, dass zur Abdichtung des Innenraums am Außenumfang des Kolbens eine umlaufende Abstreiflippe vorgesehen ist. Bevorzugt ist diese auf der der Vorderseite des Innenraums zuwandten Seite des Kolbens angeordnet.

Besonders stabil robust laufende erfindungsgemäße Druckgasmotoren können sich dadurch auszeichnen, dass der Vibrationskörper zwei unterschiedlich große Querschnittsflächen senkrecht zur linearen Bewegungsrichtung des Vibrationskörpers aufweist, die zu den Querschnittsflächen des vorderen Bereichs des Innenraums und des hinteren Bereichs des Innenraums passen, und die Querschnittsfläche auf der der Rückseite zugewandten Seite des Vibrationskörpers zumindest 100% größer ist als die Querschnittsfläche der gegenüberliegenden Vorderseite des Vibrationskörpers, bevorzugt die Querschnittsfläche auf der der Rückseite zugewandten Seite des Vibrationskörpers zumindest vier Mal so groß ist wie die Querschnittsfläche der gegenüberliegenden Vorderseite des Vibrationskörpers.

Mit der Erfindung wird auch vorgeschlagen, dass der vordere Bereich des Innenraums über ein Übergangsstück mit dem hinteren Bereich des Innenraums verbunden ist. Bevorzugt kann dabei vorgesehen sein, dass das Übergangsstück eine senkrecht zur Bewegungsrichtung des Vibrationskörpers orientierte Wandung des Innenraums ist.

Der Druckgasmotor kann ein Ventilelement zur Regulierung des Druckgasstroms in den Druckgasmotor, insbesondere durch die Gaseinlassöffnung des Druckgasmotors aufweisen. Bevorzugt ist dieses Ventilelement manuell bedienbar.

Erfindungsgemäß kann ferner vorgesehen sein, dass der Druckgasmotor, bevorzugt bis auf den Vibrationskörper und das Rückstellelement, aus thermoplastischen Kunststoffen gefertigt ist, wobei Polypropylen, Polyethylen, Polyamid-6 und Polyamid-12 besonders bevorzugt sind. Daneben sind alle technisch üblichen Kunststoffe geeignet.

Erfindungsgemäße Druckgasmotoren arbeiten bevorzugt mit einer Frequenz von 500 bis 2000 Zyklen pro Minute, besonders bevorzugt mit einer Frequenz von 1000 bis 1500 Zyklen pro Minute.

Um die Bewegung des Kolbens des Druckgasmotors mechanisch nutzbar zu machen, kann an dem Kolben, insbesondere an der von dem Vibrationskörper abgewandten Seite, eine Pleuelstange beweglich oder eine Stange fest mit dem Kolben verbunden sein. Bevorzugt kann die Stange oder Pleuelstange sich durch die Druckfeder zwischen dem Kolben und der Vorderseite des Innenraums hindurch und eine Öffnung in der Vorderseite des Innenraums erstrecken.

Die Aufgaben der Erfindung bezüglich eines Lavage-Systems werden gelöst durch ein Lavage-System aufweisend zumindest einen erfindungsgemäßen Druckgasmotor, bei dem mit dem Druckgasmotor oder den Druckgasmotoren ein periodischer Sprühstoß mit einer Flüssigkeit erzeugbar ist.

Die Aufgaben der Erfindung werden ferner gelöst durch die Verwendung eines erfindungsgemäßen Druckgasmotors als Motor für ein Lavage-System, einen Klopfermotor, einen Vibrationsmotor, als Antrieb für eine Dosiervorrichtung, als Rüttelmotor oder als Pumpe, insbesondere als Schmiermittelpumpe.

Die der Erfindung zugrundeliegenden Aufgaben werden auch gelöst durch ein Verfahren zum Erzeugen einer periodischen Bewegung mit einem Druckgas, insbesondere unter Verwendung eines erfindungsgemäßen Druckgasmotors, bei dem ein Vibrationskörper eines Druckgasmotors in Schwingung versetzt wird, indem ein Druckgas durch den Druckgasmotor geleitet wird, wobei der Vibrationskörper bei der Schwingung wiederholt auf den Kolben trifft und diesen gegen ein Rückstellelement bewegt und der Kolben von dem Rückstellelement wiederholt in eine Position überführt wird, in der der Vibrationskörper bei seiner Schwingung den Kolben trifft, und wobei die Bewegung des Kolbens als Antrieb des Druckgasmotors genutzt wird, insbesondere zum Erzeugen eines Sprühstoßes einer Flüssigkeit genutzt wird.

Die der Erfindung zugrundeliegenden Aufgaben werden auch gelöst durch ein Verfahren zum Erzeugen einer periodischen Bewegung mit einem Druckgas, insbesondere unter Verwendung eines erfindungsgemäßen Druckgasmotors, mit den Schritten:
A) in einer ersten Position eines Vibrationskörpers in einem Innenraum ein hinterer Bereich des Innenraums zwischen dem Vibrationskörper und einer Rückseite des Innenraums durch einen ersten Kanal im Vibrationskörper mit einer Gaseinlassöffnung verbunden ist und ein Druckgas durch eine Gaseinlassöffnung und den ersten Kanal in den hinteren Bereich des Innenraums zwischen dem Vibrationskörper und der Rückseite des Innenraums eingeleitet wird;
B) durch den sich zwischen dem Vibrationskörper und der Rückseite des Innenraums hinteren Bereich des Innenraums höheren Druck und dem auf der gegenüberliegenden Seite des Vibrationskörper lastenden niedrigeren Druck der Vibrationskörper in Richtung einer Vorderseite des Innenraums beschleunigt wird;
C) durch die Bewegung des Vibrationskörpers in Richtung der Vorderseite des Innenraums die Verbindung von dem hinteren Bereich des Innenraums zwischen dem Vibrationskörper und der Rückseite des Innenraums zu der Gaseinlassöffnung getrennt wird;
D) der Vibrationskörper durch die Bewegung in Richtung der Vorderseite des Innenraums mit einem vorderen Teil des Vibrationskörpers in einem vorderen Bereich des Innenraums auf einen Kolben trifft und diesen in Richtung der Vorderseite des Innenraums beschleunigt wird, wobei ein Rückstellelement durch die Auslenkung des Kolbens Energie aufnimmt und speichert;
E) durch die Bewegung des Vibrationskörpers in Richtung der Vorderseite des Innenraums der hintere Bereich des Innenraums zwischen dem Vibrationskörper und der Rückseite des Innenraums durch den ersten Kanal oder einen zweiten Kanal im Vibrationskörper mit einer Gasauslassöffnung verbunden wird;
F) das Gas aus dem hinteren Bereich des Innenraums zwischen dem Vibrationskörper und der Rückseite des Innenraums durch den ersten Kanal oder den zweiten Kanal und durch die Gasauslassöffnung ausströmt, bevorzugt an die Umgebung abgegeben wird;
G) der Kolben durch die Abgabe der Energie des Rückstellelements in Richtung der Rückseite des Innenraums beschleunigt wird;
H) der Vibrationskörper durch das Auftreffen des Kolbens und/oder durch eine Gasfeder und/oder ein zweites Rückstellelement in Richtung der Rückseite des Innenraums beschleunigt wird;
I) durch die umgekehrte Bewegung des Vibrationskörpers in Richtung der Rückseite des Innenraums die Verbindung von dem hinteren Bereich des Innenraums zwischen dem Vibrationskörper und der Rückseite des Innenraums zu der Gasauslassöffnung getrennt wird; und
J) durch die umgekehrte Bewegung des Vibrationskörpers in Richtung der Rückseite des Innenraums der hintere Bereich des Innenraums zwischen dem Vibrationskörper und der Rückseite des Innenraums durch den ersten Kanal mit der Gaseinlassöffnung verbunden wird und der Vibrationskörper in die erste Position überführt wird.

Die Schritte finden bevorzugt in der logischen beziehungsweise chronologischen Reihenfolge statt, wobei sich die Zeiträume, zu denen die erfindungsgemäßen Schritte stattfinden, teilweise und zeitweise überlagern.

Bei erfindungsgemäßen Verfahren kann vorgesehen sein, dass der Zyklus sich mit dem erneuten Einspeisen des Druckgases in den hinteren Bereich des Innenraums zwischen dem Vibrationskörper und der Rückseite des Innenraums wiederholt.

Des Weiteren kann vorgesehen sein, dass der der Vorderseite des Innenraums zugewandte Teil des hinteren Bereichs des Innenraums, der auf der der Rückseite zugewandten Seite dieses Teils des Innenraums durch den hinteren Teil des Vibrationskörpers verschlossen ist, als Gasfeder zum Beschleunigen des Vibrationskörpers in Richtung der Rückseite des Innenraums verwendet wird.

Es kann bei erfindungsgemäßen Verfahren ferner vorgesehen sein, dass die periodische, lineare Bewegung des Vibrationskörpers im Druckgasmotor selbständig durch Einwirkung von Druckgas ohne Einwirkung äußerer Ventile ausgelöst wird.

Erfindungsgemäße Verfahren werden bevorzugt mit einer Frequenz von 500 bis 2000 Zyklen pro Minute wiederholt, besonders bevorzugt mit einer Frequenz von 1000 bis 1500 Zyklen pro Minute wiederholt.

Die der Erfindung zugrundeliegenden Aufgaben werden auch gelöst durch ein Verfahren zum Erzeugen eines Sprühstoßes aufweisend die genannten erfindungsgemäßen Verfahrensschritte, wobei bei einer Bewegung des Kolbens von der Rückseite des Innenraums weg eine Spülflüssigkeit oder ein Flüssigkeit-Gas-Gemisch aus dem vorderen Bereich des Innenraums zwischen dem Kolben und der Vorderseite des Innenraums durch eine Flüssigkeitsauslassöffnung an der Vorderseite des Innenraums hinaus gepresst wird und bei einer Bewegung des Kolbens auf die Rückseite des Innenraums zu eine Flüssigkeit oder ein Flüssigkeit-Gas-Gemisch in den vorderen Bereich des Innenraums zwischen dem Kolben und der Vorderseite des Innenraums durch eine Flüssigkeitseinlassöffnung hinein gedrückt oder gezogen wird.

Bei diesem Verfahren kann vorgesehen sein, dass bei der Bewegung des Kolbens auf die Vorderseite des Innenraums zu durch den Druck in dem vorderen Bereich des Innenraums zwischen dem Kolben und der Vorderseite des Innenraums ein Ventil an der Flüssigkeitsauslassöffnung geöffnet wird und/oder offen gehalten wird und ein an die Flüssigkeitseinlassöffnung angeschlossenes Ventil geschlossen wird und/oder geschlossen gehalten wird und bei der Bewegung des Kolbens auf die Rückseite des Innenraums zu durch den geringeren Druck in dem vorderen Bereich des Innenraums zwischen dem Kolben und der Vorderseite des Innenraums das Ventil an der Flüssigkeitsauslassöffnung geschlossen wird und/oder geschlossen gehalten wird und das an die Flüssigkeitseinlassöffnung angeschlossene Ventil geöffnet wird und/oder offen gehalten wird.

Der Erfindung liegt die überraschende Erkenntnis zugrunde, dass es durch den erfindungsgemäßen Aufbau gelingt, den Kolben mit dem Vibrationskörper periodisch anzustoßen und damit eine stabil laufende periodische Bewegung des Kolbens mit hoher Frequenz zu ermöglichen. Dabei ist der Aufbau sehr einfach, kostengünstig zu realisieren und unanfällig für Störungen. Durch Druckgas angetriebene Vibratoren basieren dabei bevorzugt auf einer durch ein Druckgas verursachten Schwingung des Vibrationskörpers gegen ein Gaspolster und/oder eine zweites Rückstellelement.

Ein wichtiger Vorteil des erfindungsgemäßen Druckgasmotors gegenüber herkömmlichen Druckgasmotoren liegt darin, dass die Schwingung des Kolbens, der die Arbeit verrichten soll, und insbesondere die Frequenz der Schwingung des Kolbens, unabhängig von der Schwingung des Vibrationskörpers ist, an dem das Druckgas die Arbeit leistet. Dadurch kann der Kolben mit einer anderen, insbesondere mit einer höheren Frequenz laufen als der Vibrationskörper. Hierdurch kann bei Lavage-Systemen eine wesentlich höhere (zweifache, dreifache, vierfache oder mehrfache) Pumpfrequenz erzielt werden, als wenn die Pumpleistung unmittelbar von dem Vibrationskörper angetrieben würde. Dies gilt selbstverständlich auch für andere periodische Antriebe, also bei anderen Anwendungen des Druckgasmotors als bei Pumpen. Bei dieser Anwendung ist kein Getriebe zur Veränderung der Arbeits-Frequenz des Kolbens notwendig. Im Gegenzug leidet darunter die Kraft jeder einzelnen Schwingung und je nach Sorgfalt beim Aufbau des Druckgasmotors die Genauigkeit der Arbeitsfrequenz. Bei Anwendungen, bei denen es nicht so genau hierauf ankommt, kann diese Möglichkeit jedoch gut genutzt werden.

Die Erfindung beruht auf der Idee, einen durch Druckgas angetriebenen axial frei schwingenden Vibrationskörper auf einen ebenfalls axial beweglichen Kolben einwirken zu lassen, der mit einem Rückstellelement, wie einem axial verformbaren Federelement, verbunden ist. Der Kolben bildet mit dem Rückstellelement, beziehungsweise mit der Feder ein zweites schwingungsfähiges System. Dieses wird bei Kontakt mit dem Vibrationskörper in Schwingung versetzt. Der Vibrationskörper überträgt durch Stöße auf den Kolben Impulse, wodurch sich der Kolben gegen das Rückstellelement bewegt. Das elastische Federelement wird dabei elastisch verformt beziehungsweise das Rückstellelement nimmt Energie auf. Anschließend expandiert das Federelement und schiebt den Kolben in die Ausgangslage zurück. Der Kolben bildet mit dem Federelement ein axial schwingendes System.

Der Druckgasmotor kann die in vielen Bereichen, insbesondere in Operationssälen, ohnehin vorhandenen Druckgasquellen nutzen, um den Druckgasmotor beziehungsweise den Vibrationskörper anzutreiben. Es wird dann keine zusätzliche Energiequelle zum Antreiben des Druckgasmotors benötigt. Gleichzeitig ist in solchen Fällen das Druckgas in praktisch unbegrenzter Menge vorhanden. Alternativ kann der Druckgasmotor auch mit Druckgaspatronen, wie beispielsweise CO₂-Patronen betrieben werden.

Mit dem erfindungsgemäßen Lavage-System oder Spülsystem ist es möglich, gepulste Flüssigkeitsstrahlen zu erzeugen, wobei die Pulsrate bei 1000 bis 2500 Pulsen pro Minute liegt.

Ein erfindungsgemäßes medizinisches Spülsystem (Lavage-System) kann beispielsweise zusammengesetzt sein aus
a) einem ersten Hohlzylinder (hinterer Bereich des Innenraums) mit einer Gaseinlassöffnung,
b) einem zweiten Hohlzylinder (vorderer Bereich des Innenraums) mit einer Gasauslassöffnung, einer Flüssigkeitseinlassöffnung und einer Flüssigkeitsauslassöffnung, wobei der zweite Holzylinder einen geringeren Innendurchmesser als der erste Hohlzylinder hat,
c) einem Übergangsstück, das den ersten Hohlzylinder mit dem zweiten Hohlzylinder verbindet,
d) einem Vibrationskörper mit einer Dichte größer 4 g/cm³, der aus einem ersten zylindrischen Teil (hinterer Teil des Vibrationskörpers) besteht, das im ersten Hohlzylinder axial beweglich angeordnet ist, und einem zweiten zylindrischen Teil (vorderer Teil des Vibrationskörpers), das im zweiten Hohlzylinder axial beweglich angeordnet ist, wobei der Außendurchmesser des ersten Teils des Vibrationskörpers kleiner als der Innendurchmesser des ersten Hohlzylinders ist und der Außendurchmesser des zweiten Teils des Vibrationskörpers kleiner als der Innendurchmesser des zweiten Hohlzylinders ist,
e) einem im zweiten Hohlzylinder axial beweglich angeordneten Kolben,
f) einem Federelement, das zwischen der Schmalseite des zweiten Hohlzylinders und dem axial beweglichen Kolben angeordnet ist,
g) einer Druckgaszuführung,
h) einem Ventilelement zu Regulierung des Druckgasstroms,
i) einer Flüssigkeitszuführung, die mit der Flüssigkeitseinlassöffnung verbunden ist,
j) einem Flüssigkeitsaustragsrohr, das mit der Flüssigkeitsauslassöffnung verbunden ist, und wobei der Abstand zwischen dem Kolben, bei maximaler Ausdehnung des Federelements, und dem Vibrationskörper, bei maximaler Auslenkung des Vibrationskörpers in Richtung des Kolbens, so ist, dass eine Berührung des Kolbens durch den Vibrationskörper erfolgt, und der Kolben in axiale Schwingung versetzt wird, ohne dass der Vibrationskörper gestoppt wird.

Bei erfindungsgemäßen Lavage-Systemen beziehungsweise Druckgasmotoren zum Ausstoßen einer Flüssigkeit kann vorgesehen sein, dass die dem Vibrationskörper entgegengesetzte Stirnseite des Kolbens mit dem zweiten Hohlzylinder eine Pumpe bildet.

Dazu sind im zweiten Hohlzylinder eine Flüssigkeitseinlassöffnung und eine Flüssigkeitsauslassöffnung angeordnet. Die Flüssigkeitseinlassöffnung ist mit einem Ventil mit Richtwirkung verbunden, zum Beispiel mit einem Lippenventil. Das bedeutet, das Ventil öffnet nur bei einem Flüssigkeitsstrom in Richtung des Hohlzylinders. Bei der Flüssigkeitsauslassöffnung ist ebenfalls ein Ventil mit Richtwirkung eingesetzt, das jedoch umgekehrt nur bei einem aus dem zweiten Hohlzylinder heraus führenden Flüssigkeitsstrom öffnet. In dem durch den zweiten Hohlzylinder und der Stirnseite des Kolbens gebildeten Raum befindet sich das Federelement, das sich an der Stirnseite des Hohlzylinders (Vorderseite des Innenraums) abstützt. Bei axialer Schwingung des Kolbens vergrößert und verringert sich periodisch das Volumen des Hohlraums, des durch den Hohlzylinder und der Stirnfläche des Kolbens gebildeten Hohlraums. Dadurch entstehen periodisch ein Über- und ein Unterdruck. Bei Unterdruck wird Flüssigkeit über die Flüssigkeitseintrittsöffnung angesaugt und bei Überdruck wird die Flüssigkeit aus dem Hohlraum über die Flüssigkeitsauslassöffnung ausgepresst.

Vor der Gaseinlassöffnung des Druckgasmotors kann ein Sterilfilter angeordnet sein, der bei Verwendung von unsterilem Druckgas dieses filtriert, um eine mikrobielle Kontamination des OP-Felds zu verhindern. Es ist ebenso möglich, nach der Gasauslassöffnung einen Sterilfilter anzuordnen, der das zumindest teilentspannte Druckgas steril filtriert.

Für die vorliegende Erfindung ist es wichtig, dass der Kolben so angeordnet ist, dass der Vibrationskörper bei seiner axialen Schwingung einen Impuls auf den Kolben übertragen kann, jedoch ohne dass der Vibrationskörper stehen bleibt. Es zeigte sich, dass eine maximale Auslenkung des Kolbens von 3 mm möglich ist, ohne dass der Vibrationskörper stehen bleibt. Erfindungsgemäß kann daher vorgesehen sein, dass der Vibrationskörper den Kolben um maximal 3 Millimeter in Richtung der Vorderseite des Innenraums beziehungsweise in Richtung der Feder verschiebt.

Es wurde im Rahmen der vorliegenden Erfindung ferner gefunden, dass gummielastische Dichtungsringe zur Abdichtung des Raums zwischen der Innenseite des vorderen Bereichs des Innenraums und dem Außendurchmesser des Kolbens ungeeignet sind, weil diese auf Grund ihrer Reibung an der Innenseite des vorderen Bereichs des Innenraums nur geringe Pulszahlen zulassen. Zur Abdichtung des Raums zwischen der Innenseite des vorderen Bereichs des Innenraums und dem Außendurchmesser des Kolbens ist deshalb bevorzugt eine Abstreiflippe angeordnet. Diese Abstreiflippe übt einen geringeren Reibungswiderstand aus als üblicher Dichtungsringe. Dadurch sind Pulszahlen von mehr als 1000 Pulsen pro Minute möglich.

Ebenso ist es für die vorliegende Erfindung wichtig, dass der Vibrationskörper eine Dichte bevorzugt von größer 4 g/cm³ und ganz besonders bevorzugt von größer 7 g/cm³ hat. Die hohe Dichte des Vibrationskörpers ist notwendig, um einen großen Impuls auf den Kolben übertragen zu können. Der Vibrationskörper kann aus Kunststoff großer Dichte, zum Beispiel mit Schwerspat oder mit Wolfram gefüllter Kunststoff, oder auch aus Stahl oder aus Messing oder anderen Metalllegierungen gefertigt sein.

Der Vibrationskörper besitzt bevorzugt mindestens einen gasdurchlässigen Kanal, der sich von der dem Kolben entgegen gesetzten Längsseite in Richtung des Kolbens erstreckt, wobei wenigstens zwei gasdurchlässige Kanäle mit dem Längskanal und der Mantelfläche des Vibrationskörpers verbunden sind und diese Kanäle entlang der Längsachse des Vibrationskörper versetzt angeordnet sind.

Im Folgenden werden weitere Ausführungsbeispiele der Erfindung anhand von sechs schematisch dargestellten Figuren erläutert, ohne jedoch dabei die Erfindung zu beschränken. Dabei zeigt:
- Figur 1:: eine schematische Querschnittansicht eines erfindungsgemäßen Druckgasmotors mit einem Vibrationskörper, bei dem die Gaseinlassöffnung geöffnet mit der Rückseite des Innenraums verbunden ist;
- Figur 2:: eine schematische Querschnittansicht des erfindungsgemäßen Druckgasmotors nach Figur 1, bei dem der Vibrationskörper durch das Druckgas oder die Druckdifferenz auf den Kolben stößt und die Gasauslassöffnung mit der Rückseite des Innenraums verbunden ist;
- Figur 3:: eine schematische Querschnittansicht des erfindungsgemäßen Druckgasmotors nach Figur 1, bei dem der Kolben zur Vorderseite bewegt ist und der Vibrationskörper sich in einer Position befindet, bei der die Gaseinlassöffnung und die Gasauslassöffnung geschlossen sind;
- Figur 4:: eine schematische Querschnittansicht des erfindungsgemäßen Druckgasmotors nach Figur 1, bei dem der Vibrationskörper fast an der Rückseite des Innenraums anliegt und Druckgas in den hinteren Bereich des Innenraums strömt;
- Figur 5:: eine schematische Querschnittansicht des alternativen erfindungsgemäßen Druckgasmotors mit zwei Kanälen; und
- Figur 6:: eine schematische Querschnittansicht eines erfindungsgemäßen Lavage-Systems mit einem erfindungsgemäßen Druckgasmotor.

In den Figuren werden teilweise für gleiche oder gleichartige Bauteile die gleichen Bezugszeichen verwendet, auch wenn es sich um unterschiedliche Druckgasmotoren handelt.

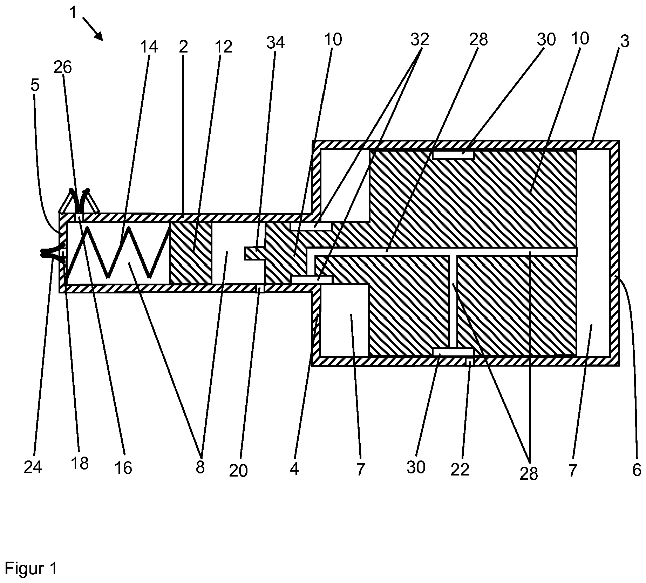

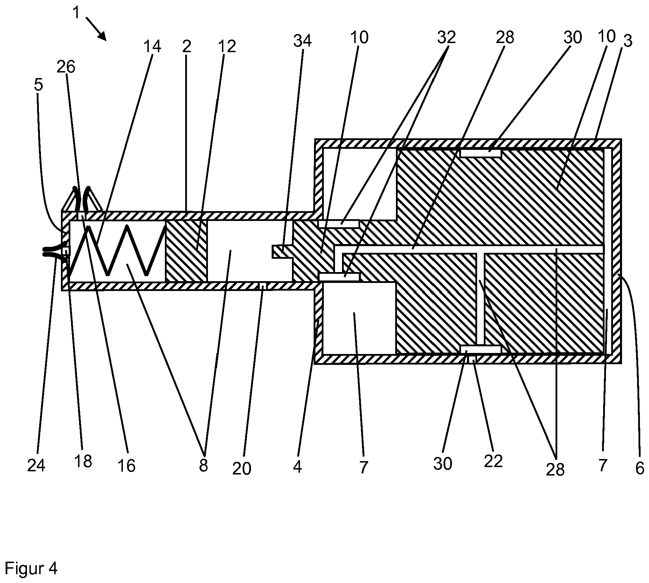

Die Figuren 1 bis 4 zeigen schematische Querschnittansichten eines erfindungsgemäßen Druckgasmotors 1 während eines Arbeitszyklus in chronologischer Abfolge. Die Querschnitte enthalten die Symmetrieachse der Bauteile des Druckgasmotors 1, das heißt, der Querschnitt ist mittig geschnitten dargestellt. Der Druckgasmotor 1 weist ein Gehäuse 2, 3, 4, 5, 6 aus Kunststoff mit einem zweiteiligen zylindrischen Innenraum 7, 8 auf. Das Gehäuse 2, 3, 4, 5, 6 beziehungsweise der Innenraum 7, 8 ist auf der Vorderseite 5 durch eine Deckplatte 5 und auf der Rückseite 6 durch eine Rückplatte 6 geschlossen. Die Deckplatte 5 und die Rückplatte 6 sind kreisförmige Scheiben. Der vordere Bereich 8 des zylindrischen Innenraums ist an der Mantelfläche durch die vordere Wandung 2 begrenzt, die durch ein zylindrisches Rohr aufgebaut ist. Der hintere Bereich 7 des zylindrischen Innenraums ist an der Mantelfläche durch die hintere Wandung 3 begrenzt, die ebenfalls durch ein zylindrisches Rohr aufgebaut ist. Der hintere Bereich 7 des Innenraums hat einen etwa dreimal größeren Durchmesser als der vordere Bereich 8 des Innenraums. Der Übergang von dem vorderen Bereich 8 des Innenraums zum hinteren Bereich 7 des Innenraums wird durch eine Übergangswandung 4 gebildet, die die vordere Wandung 2 mit der hinteren Wandung 3 verbindet und die parallel zu der Deckplatte 5 und der Rückplatte 6 beziehungsweise parallel zu der Vorderseite 5 und der Rückseite 6 des zylindrischen Innenraums 7, 8 angeordnet ist. Die Übergangswandung 4 ist als Kreisscheibe mit einer zentralen kreisförmigen Ausnehmung ausgebildet. Die zentrale kreisförmige Ausnehmung beziehungsweise die Ebene der Übergangswandung 4 bildet die Grenzfläche zwischen dem vorderen Bereich 8 des Innenraums und dem hinteren Bereich 7 des Innenraums. Die zentrale kreisförmige Ausnehmung hat den gleichen Durchmesser wie der vordere Bereich 8 des Innenraums.

Im Inneren des Innenraums 7, 8 ist ein Vibrationskörper 10 angeordnet. Der Vibrationskörper 10 ist aus Messing gefertigt und hat die Form zweier über die Grundflächen entlang der Symmetrieachsen aneinandergesetzter Zylinder. Der Vibrationskörper 10 ist entlang seiner Zylindersymmetrieachse in Richtung der Vorderseite 5 und der Rückseite 6 des Innenraums 7, 8 linear verschiebbar im Innenraum 7, 8 angeordnet. Der vordere Teil des Vibrationskörpers 10, der der Vorderseite 5 zugewandt ist, passt mit seinem Außenumfang in den Innenumfang des vorderen Bereichs 8 des Innenraums. Ebenso passt der hintere Teil des Vibrationskörpers 10, der der Rückseite 5 zugewandt ist, mit seinem Außenumfang in den Innenumfang des hinteren Bereichs 7 des Innenraums. Der vordere Teil des Vibrationskörpers 10 kann sich somit auch in dem vorderen Bereich 8 des Innenraums bewegen.

Zwischen der Vorderseite 5 und dem Vibrationskörper 10 ist im vorderen Bereich 8 des Innenraums ein Kolben 12 aus einem festen und mechanisch widerstandfähigen Kunststoff angeordnet, der bei dem gezeigten Druckgasmotor 1 als Pumpkolben 12 verwendet wird. Der Kolben 12 hat eine zylindrische Form, so dass die Mantelfläche des Kolbens 12 an den Innenwänden des Gehäuses 2 anliegt. Der Kolben 12 ist mit einer Druckfeder 14 aus Stahl oder einem Kunststoff mit geeigneter Elastizität schwingungsfähig in dem vorderen Bereich 8 des Innenraums gelagert. Dazu ist die Druckfeder 14 in dem vorderen Bereich 8 des Innenraums zwischen dem Kolben 12 und der Vorderseite 5 angeordnet und liegt am Kolben 12 und der Vorderseite 5 an.

In der Gehäusewandung 2 des Zylindermantels des vorderen Bereichs 8 des Innenraums ist eine Flüssigkeitseinlassöffnung 16 vorgesehen. Die Flüssigkeitseinlassöffnung 16 ist derart positioniert, dass der Kolben 12 in keiner Stellung im Betrieb des Druckgasmotors 1 zwischen der Flüssigkeitseinlassöffnung 16 und der Vorderseite 5 des Innenraums angeordnet sein kann. Dadurch soll sichergestellt werden, dass keine Flüssigkeit in den Zwischenraum 8 zwischen den Kolben 12 und dem Vibrationskörper 10 gelangen kann. Damit der Pumpraum 8 zwischen dem Kolben 12 und der Vorderseite 5 möglichst leicht und vollständig mit Flüssigkeit gefüllt werden kann, ist die Flüssigkeitseinlassöffnung 16 möglichst dicht bei der Vorderseite 5 des Innenraums 8 angeordnet. Die Flüssigkeitseinlassöffnung 16 kann auch in der Vorderseite 5 angeordnet sein.

In der Vorderseite 5 ist eine Flüssigkeitsauslassöffnung 18 zum Austreiben eines Flüssigkeitsstrahls vorgesehen. Der Druckgasmotor 1 ist also zum Erzeugen eines gepulsten Strahls einer Spülflüssigkeit aus der Flüssigkeitsauslassöffnung 18 geeignet und vorgesehen.

In der Seitenwand 2 des vorderen Bereichs 8 des Innenraums ist eine Gasauslassöffnung 20 vorgesehen, durch die das in den Druckgasmotor 1 eingespeiste Druckgas und im Druckgasmotor 1 entspannte Druckgas wieder entweichen kann. Die Gasauslassöffnung 20 ist dazu zwischen dem Kolben 12 in seiner maximalen Auslenkung in Richtung Rückseite 6 beziehungsweise in Richtung des Vibrationsköpers 10 und der Ebene der Übergangswandung 4 angeordnet. Hierdurch soll sichergestellt werden, dass die Gasauslassöffnung 20 von dem Vibrationskörper 10 verdeckt ist oder zwischen dem Vibrationskörper 10 und dem Kolben 12 angeordnet ist und dann den vorderen Bereich 8 des Innenraums dazwischen mit der Umgebung verbindet.

In der Zylindermantelfläche des hinteren Bereichs 7 des Innenraums ist eine Gaseinlassöffnung 22 vorgesehen, durch die das Druckgas in den Druckgasmotor 1 eingespeist wird.

Der hintere Bereich 7 des Innenraums ist im Durchmesser senkrecht zur Zylinderachse etwa dreimal so groß wie die Durchmesser des vorderen Bereichs 8 des Innenraums und damit in der Querschnittsfläche etwa neunmal so groß wie die Querschnittsfläche des vorderen Bereichs 8 des Innenraums. Dadurch weist der hintere Innenraum 7 eine vordere Vorderwand 4 (die Übergangswandung 4) und die hintere (rückseitige) Rückwand 6 auf, die die Wandungen 3 des hinteren Innenraums mit den Wandungen 2 des vorderen Innenraums 8 verbinden. Der hintere Teil des Vibrationskörpers 10 kann von der Übergangswandung 4 des hinteren Innenraums 7 mit mehreren Abstandhaltern (nicht gezeigt) beabstandet sein, die dafür sorgen, dass der Vibrationskörper 10 nicht an der Übergangswandung 4 des hinteren Innenraums 7 anliegen kann. Da das Gas in dem Zwischenraum 7, zwischen dem hinteren Teil des Vibrationskörpers 10 und der Übergangswandung 4 nicht oder zumindest nicht sehr schnell entweichen kann und daher bei einer Bewegung des Vibrationskörpers 10 auf die Übergangswandung 4 zu komprimiert wird, wirkt dieser Zwischenraum 7 als Gasfederung. Daher sind Abstandhalter auch nicht wirklich notwendig.

Vor der Flüssigkeitsauslassöffnung 18 ist ein Lippenventil 24 angeordnet, mit der die Flüssigkeitsauslassöffnung 18 geschlossen wird, wenn in dem vorderen Bereich 8 des Innenraums zwischen der Vorderseite 5 und dem Kolben 12 ein geringer Druck vorliegt. Das Lippenventil 24 öffnet sich, wenn eine Flüssigkeit aus dem vorderen Innenraum 8 ausgestoßen wird, das heißt, wenn der Druck im vorderen Bereich 8 des Innenraums zwischen dem Kolben 12 und der Vorderseite 5 ausreichend groß ist. Das heißt, dass der Druck im vorderen Bereich 8 des Innenraums größer ist als der Umgebungsdruck und die elastische Kraft des Lippenventils 24.

Vor der Flüssigkeitseinlassöffnung 16 ist ebenfalls ein Lippenventil 26 angeordnet, so dass Flüssigkeit nur dann in den vorderen Bereich 8 des Innenraums eingesaugt werden kann, wenn in dem vorderen Bereich 8 des Innenraums ein ausreichend niedriger Druck vorliegt. Dieser wird dann erreicht, wenn sich der Kolben 12 in Richtung des Vibrationskörpers 10 bewegt und dadurch das Lippenventil 24 vor der Flüssigkeitsauslassöffnung 18 geschlossen ist. Wenn sich das Lippenventil 26 öffnet, strömt im Betrieb des Druckgasmotors 1 als Pumpeinrichtung eine Flüssigkeit durch die Flüssigkeitseinlassöffnung 16 in der vorderen Bereich 8 des Innenraums.

Die rückseitige Wandung 6 schließt den rückseitigen also hinteren Bereich 7 des Innenraums zwischen dem Vibrationskörper 10 und der Rückseite 6 gasdicht und druckdicht ab. Dieser rückseitige Raum 7 kann als Arbeitsraum 7 bezeichnet werden.

In dem Vibrationskörper 10 ist ein Kanal 28 in Form einer T-Stücks vorgesehen. Zudem ist in den seitlichen Zylindermänteln der beiden zylindrischen Teile des Vibrationskörpers 10 jeweils eine umlaufende Nut 30, 32 vorgesehen. Das T-Stück 28 mündet in die rückseitige Grundfläche des Vibrationskörpers 10 sowie in die beiden Nuten 30, 32. Der Kanal 28 kann eine zentrale Bohrung entlang der Zylinderachse des Vibrationskörpers 10 und mehrere oder eine Vielzahl radialer Bohrungen aufweisen, die die zentrale Bohrung mit den Nuten 30, 32 in dem Zylindermantel der beiden zylindrischen Teile des Vibrationskörpers 10 verbinden. Die umlaufende Nut 30 in dem Zylindermantel mit dem größeren Durchmesser, also in dem hinteren Teil des Vibrationskörpers 10, dient der Verbindung des Kanals 28 mit der Gaseinlassöffnung 22. Die umlaufende Nut 32 in dem Zylindermantel mit dem kleineren Durchmesser, also in dem vorderen Teil des Vibrationskörpers 10, dient der Verbindung des Kanals 28 mit der Gasauslassöffnung 20. Durch die umlaufenden Nuten 30, 32 kann sichergestellt werden, dass die Gaseinlassöffnung 22 und die Gasauslassöffnung 20 unabhängig von einer Rotation des Vibrationskörpers 10 um seine Symmetrieachse mit dem Kanal 28 verbunden werden können. Der Kanal 28 erstreckt sich nicht bis zur Vorderseite des Vibrationskörpers 10.

An der Vorderseite des Vibrationskörpers 10 ist ein Vorsprung 34 angeordnet, der zum Anstoßen des Kolbens 12 vorgesehen ist. Dadurch soll verhindert werden, dass das zwischen dem Kolben 12 und dem Vibrationskörper 10 eingeschlossene Gas im vorderen Bereich 8 des Innenraums den Kolben 12 frühzeitig in Richtung der Vorderseite 5 drückt und dadurch ein Auftreffen des Vibrationskolbens 10 auf den Kolben 12 und damit eine starke Impulsübertragung verhindert. Der Vorsprung 34 kann ebenso gut an der Rückseite des Kolbens 12 angeordnet sein.

Die Gaseinlassöffnung 22 und die Gasauslassöffnung 20 bilden mit den Nuten 30, 32 und dem Kanal 28 in dem gegen die Öffnungen 20, 22 beweglichen Vibrationskörper 10 die Ventile des Druckgasmotors 1, die durch die Bewegung des Vibrationskörpers 10 automatisch gesteuert werden. In dem hinteren Bereich 7 des Innenraums zwischen dem Vibrationskörper 10 und der Rückseite, wird durch den Kanal 28 des Vibrationskörpers 10 das Druckgas eingeleitet. Die Situation ist in Figur 1 gezeigt. In diesem Bereich expandiert das Druckgas, triebt den Vibrationskörper 10 in Richtung des Kolbens 12 und leistet dadurch die Arbeit.

Durch die Bewegung des Vibrationskörpers 10 wird die Gaseinlassöffnung 22 von dem Kanal 28 getrennt. Zudem wird die Verbindung zwischen dem Kanal 26 beziehungsweise der vorderen Nut 32 und dem hinteren Bereich 7 des Innenraums zwischen dem Vibrationskörper 10 und der Übergangswandung 4 getrennt und damit dieser Bereich 7 abgeschlossen. Das Druckgas in dem hinteren Bereich 7 des Innenraums zwischen dem Vibrationskörper 10 und der Rückseite 6 expandiert weiter. Währenddessen wird das Gas im hinteren Bereich 7 des Innenraums zwischen dem Vibrationskörper 10 und der Übergangswandung 4 komprimiert und nimmt Energie auf.

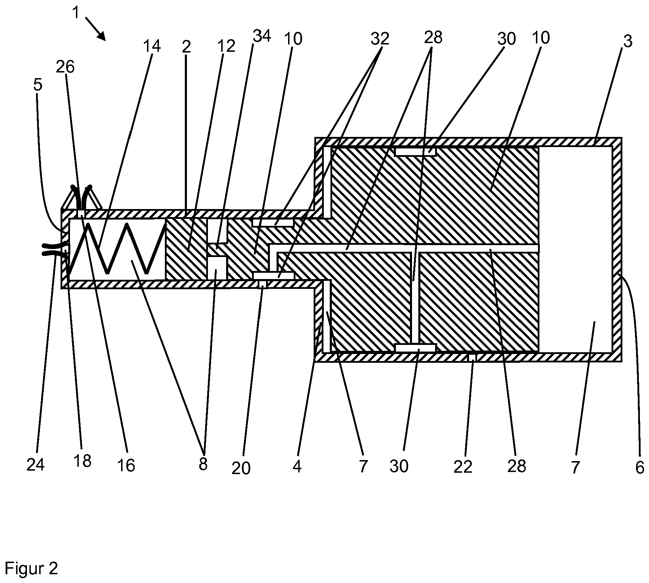

Anschließend trifft der Vibrationskörper 10 auf den Kolben 12 und überträgt seinen Impuls über den Vorsprung 34 auf den Kolben 12. Diese Situation ist in Figur 2 dargestellt.

Durch die Impulsübertragung von dem Vibrationskörper 10 auf den Kolben 12 wird der Kolben 12 in Richtung der Vorderseite 5 beschleunigt und dabei die Druckfeder 14 komprimiert. Gleichzeitig wird der Inhalt des vorderen Bereichs 8 des Innenraums zwischen dem Kolben 12 und der Vorderseite 5 durch die Flüssigkeitsauslassöffnung 18 und das Lippenventil 24 ausgestoßen. Durch den Druck, der von dem Kolben 12 auf den Inhalt des vorderen Bereichs 8 des Innenraums zwischen dem Kolben 12 und der Vorderseite 5 ausgeübt wird, bleibt das Lippenventil 26 geschlossen. Dadurch wird verhindert, dass der Inhalt zurück durch die Flüssigkeitseinlassöffnung 16 in die Flüssigkeitszufuhr gedrückt wird.

Durch die Impulsübertragung und durch die Gasfederung, das heißt durch den stark verkleinerten hinteren Bereich 7 des Innenraums zwischen dem Vibrationskörper 10 und der Übergangswand 4, wird der Vibrationskörper 10 in Richtung der Rückseite 6 des Innenraums 7, 8 beschleunigt. Da der der Rückseite 6 zugewandte Teil des hinteren Bereichs 7 des Innenraums über den Kanal 28 mit der Gasauslassöffnung 20 verbunden ist, hat sich der Druck in diesem Bereich 7 abgebaut und das expandierte Druckgas wurde über die Gasauslassöffnung 20 an die Umgebung abgegeben. Der Vibrationskörper 10 bewegt sich in Richtung der Rückseite 6 des Innenraums 7, 8. Dies führt dazu, dass die Verbindung von dem Kanal 28 zu der Gasauslassöffnung 20 wieder getrennt wird.

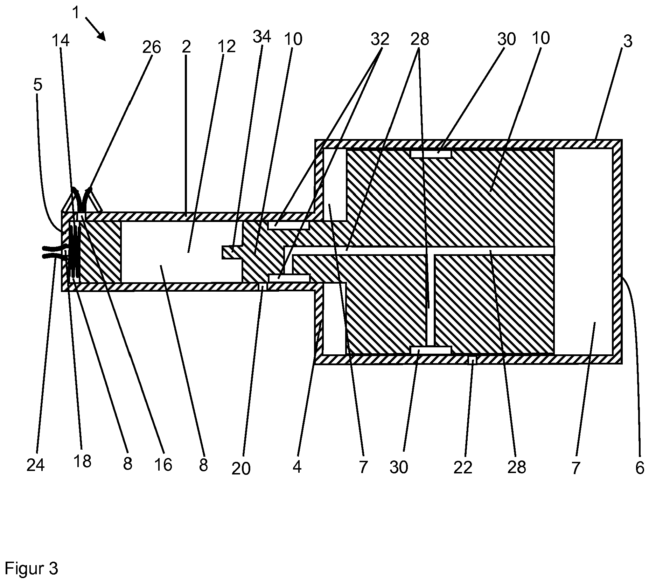

Durch das Zusammendrücken der Druckfeder 14 wird der Kolben 12 in Richtung des Vibrationskörpers 10 zurück beschleunigt. Schließlich erreicht der Kolben 12 den Wendepunkt und der Kolben 12 bewegt sich zurück in Richtung des Vibrationskörpers 10. Diese Situation ist in Figur 3 dargestellt.

Durch die Gasfederung und seine Trägheit überfährt der Vibrationskörper 10 mit der hinteren Nut 30 erneut die Gasauslassöffnung 22, so dass erneut das Druckgas durch den Kanal 28 in den hinteren Bereich 7 des Innenraums zwischen dem Vibrationskörper 10 und der Rückseite 6 strömt. Gleichzeitig strömt das Gas auch in den hinteren Bereich 7 des Innenraums zwischen dem Vibrationskörper 10 und der Übergangswand 4. Dies hat den Vorteil, dass Gasverluste in der in letzterem Bereich gebildeten Gasfeder bei jedem Zyklus ausgeglichen werden, so dass der Aufbau in diesem Bereich nicht besonders gut abgedichtet werden muss. Da die Fläche des Vibrationskörpers 10, auf die der Gasdruck in dem der Übergangswand 4 zugewandten Teil des hinteren Bereichs 7 des Innenraums lastet, kleiner ist, als der der Rückseite 6 zugewandte Teil des hinteren Bereichs 7 des Innenraums, entsteht eine Druckdifferenz zwischen den beiden Seiten des Vibrationskörpers 10, die zu einer Beschleunigung des Vibrationskörpers 10 in Richtung des Kolbens 12 führt. Die Druckdifferenz erklärt sich auch dadurch, dass auf der Vorderseite des Vibrationskörpers 10 in dem vorderen Bereich 8 des Innenraums nicht der Überdruck des Druckgases lastet sondern über die Gasauslassöffnung 20 der Umgebungsdruck, was zu der besagten Druckdifferenz über die Flächendifferenz zwischen Vorder- und Rückseite des Vibrationskörpers 10 führt.

Parallel dazu bewegt sich der Kolben 12 in Richtung des Vibrationskörpers 10. Dabei vergrößert sich der Raum des vorderen Bereichs 8 zwischen dem Kolben 12 und der Vorderseite 5. Durch den dabei entstehenden Unterdruck schließt sich das Lippenventil 24 an der Flüssigkeitsauslassöffnung 18 und das Lippenventil 26 an der Flüssigkeitseinlassöffnung 16 öffnet sich. Der Raum füllt sich dadurch erneut mit Flüssigkeit (nicht gezeigt) oder einem Flüssigkeits-Gas-Gemisch.

Wenn der Vibrationskörper 10 sich wieder in Richtung des Kolbens 12 bewegt (Figur 1) beginnt der Arbeitszyklus des Druckgasmotors 1 von vorne.

Es ist nicht notwendig, dass die Schwingung des Kolbens 12 synchron zur Schwingung des Vibrationskörpers 10 verläuft. Es reicht aus, wenn der Vibrationskörper 10 den Kolben 12 immer wieder anstößt und zum Schwingen anregt. Die Frequenz des Systems Kolben 12 und Feder 14 kann also höher sein als die Frequenz des schwingenden Vibrationskörpers 10. Die Druckfeder 14 kann dazu an der Vorderseite 5 des Innenraums 8 und am Kolben 12 befestigt sein. Die Druckfeder 14 wirkt dann bei der Schwingung des Kolbens 12 auch als Zugfeder auf den Kolben 12. Hierdurch kann eine wesentlich höhere Frequenz des Kolbens 12 erreicht werden, als es der Vibrationskörper 10 oder ein herkömmlicher Druckgasmotor erreichen könnte.

Es kann beispielsweise vorgesehen sein, dass der Kolben 12 mit einem ganzzahligen Vielfachen der Frequenz des Vibrationskörpers 10 schwingt. Der Kolben 12 beispielsweise bei jeder zweiten oder dritten Schwingung von dem Vibrationskörper 10 angestoßen wird. Frequenzabweichungen spielen dabei keine große Rolle, da die Impulsübertragung des dichteren und schwereren Vibrationskörpers 10 der Schwingung des Kolbens 12 im Zweifel seine Eigenfrequenz aufzwingt. Jedes rationale oder auch irrationale Verhältnis zwischen den Frequenzen der Schwingungen kann so zur Realisierung eines erfindungsgemäßen Druckgasmotors 1 verwendet werden.

Wenn die Feder 14 nicht an dem Kolben 12 und der Vorderseite 5 befestigt ist, stößt der Kolben 12 mit seiner der Rückseite 6 zugewandten Fläche irgendwann auf den Vibrationskörper 10. Je nachdem, in welche Richtung sich der Vibrationskörper 10 dann gerade bewegt, prallt er unterschiedlich stark von dem Vibrationskörper 10 ab und nimmt einen Impuls von dem Vibrationskörper 10 in Richtung der Vorderseite 5 auf, der die Schwingung des Kolbens 12 antreibt.

Selbst wenn also der Druckgasmotor 1 nicht mit Sorgfalt aufgebaut wird, also die Eigenfrequenzen des Kolbens 12 und des Vibrationskörpers 10 nicht aufeinander abgestimmt werden, wird der Druckgasmotor 1 noch gut funktionieren. Der Druckgasmotor 1 kann dadurch auch mit Druckgasen unterschiedlichen Drucks betrieben werden.

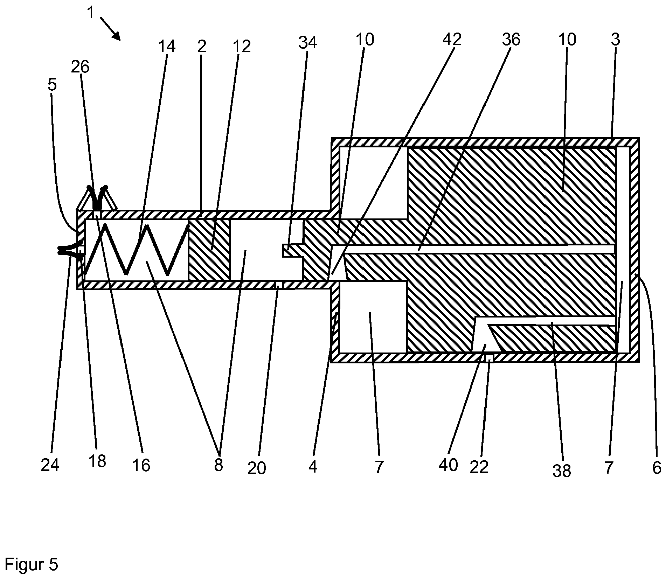

Figur 5 zeigt eine schematische Querschnittansicht des alternativen erfindungsgemäßen Druckgasmotors 1. Der Aufbau dieses Druckgasmotors 1 gleicht dem nach den Figuren 1 bis 4, bis auf den Kanal 28, die Nuten 30, 32 und die Symmetrie des Innenraums 7, 8, des Kolbens 12 und des Vibrationskörpers 10 (siehe Figuren 1 bis 4). Anstatt eines als T-Stück geformten Kanals sind im Inneren des Vibrationskörpers 10 nach Figur 5 zwei Kanäle 36, 38 vorgesehen. Der erste Kanal 36 dient der Verbindung des hinteren Bereichs 7 des Innenraums zwischen dem Vibrationskolben 10 und der geschlossenen Rückseite 6 mit dem hinteren Bereich 7 des Innenraums zwischen dem Vibrationskörper 10 und der Übergangswandung 4 oder mit der Gasauslassöffnung 20. Der zweite Kanal 38 dient der Verbindung des hinteren Bereichs 7 des Innenraums zwischen dem Vibrationskolben 10 und der geschlossenen Rückseite 6 mit der Gaseinlassöffnung 22.

Die Formen des Innenraums 7, 8, des Vibrationskörpers 10 und des Kolbens 12 sind passend zueinander gewählt bei dieser Ausführungsform aber nicht rotationssymmetrisch. Bevorzugt wird eine zylindrische Form mit nicht kreissymmetrischer aber abgerundeter Grundfläche, beispielsweise mit ovaler Grundfläche. Dies ist in der Herstellung etwas aufwendiger. Dadurch kann aber eine Drehung des Vibrationskörpers 10 in dem hinteren Bereich 7 des Innenraums verhindert werden. Genaugenommen reicht es aus, wenn nur der hintere Bereich 7 des Innenraums und der hintere Teil des Vibrationskörpers 10 nicht rotationssymmetrisch sind. Der Kolben 12 und der vordere Bereich 8 des Innenraums können dann auch kreiszylindrische Symmetrie haben.

Da sich der Vibrationskörper 10 bei dieser Ausgestaltung nicht mehr in dem Innenraum 7, 8 drehen kann, kann durch seitliche Bohrungen in den Mantelflächen des Vibrationskörpers 10 erreicht werden, dass die Mündungen 40, 42 der Kanäle 36, 38 die Gaseinlassöffnung 22 und die Gasauslassöffnung 20 treffen. Um während einer Schwingung einen Gasaustausch über etwas größere Zeiträume zu ermöglichen, sind die Mündungen 40, 42 in Längsrichtung verbreitert. Dies ist für die Funktion des Druckgasmotors 1 aber nicht notwendig.

Das Funktionsprinzip und der Arbeitszyklus des Druckgasmotors 1 nach Figur 5 gleicht denen des Druckgasmotors 1 nach den Figuren 1 bis 4.

Anstatt eine Flüssigkeit durch die Flüssigkeitsauslassöffnung 18 auszustoßen, kann bei den Druckgasmotoren 1 nach den Figuren 1 bis 5 auch eine Stange (nicht gezeigt) fest oder eine Pleuelstange (nicht gezeigt) über ein Gelenk an der Vorderseite des Kolbens 12 befestigt sein, die sich vorne aus dem Druckgasmotor 1 heraus erstrecken. Beispielsweise kann sich die Stange oder Pleuelstange durch eine zentrale Öffnung ähnlich der Flüssigkeitsauslassöffnung 18 aus dem Druckgasmotor 1 heraus erstrecken. Die Stange oder Pleuelstange kann beispielsweise als Antrieb für ein Schwungrad oder als Rüttelvorrichtung verwendet werden.

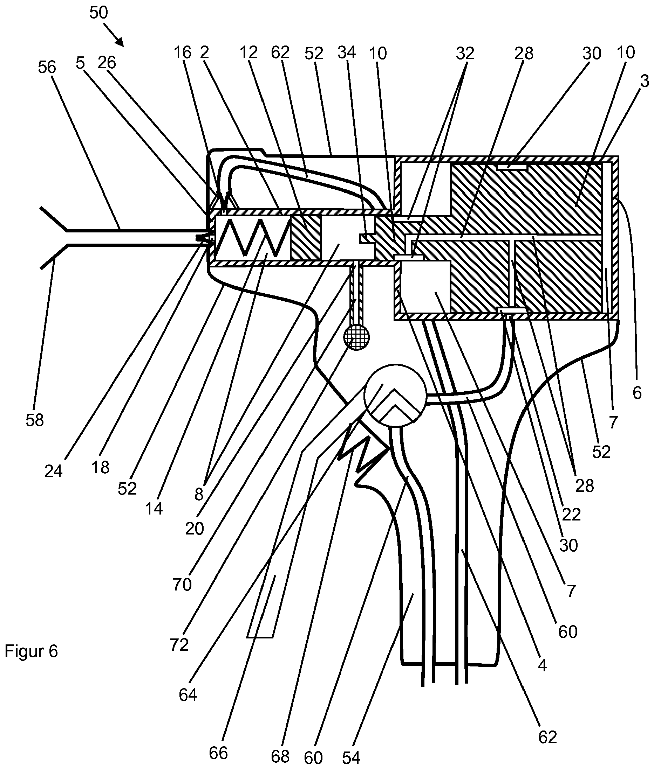

Figur 6 zeigt eine schematische Querschnittansicht eines erfindungsgemäßen, in einer Hand haltbaren Lavage-Systems 50 mit einem erfindungsgemäßen Druckgasmotor, wie er in den Figuren 1 bis 4 gezeigt ist. Da der Druckgasmotor wie der Druckgasmotor 1 nach den Figuren 1 bis 4 aufgebaut ist, kann für diesen Aufbau auf dieses Ausführungsbeispiel verwiesen werden kann. Alternativ könnte natürlich auch der Druckgasmotor 1 nach Figur 5 eingebaut werden oder ein anderer erfindungsgemäßer Druckgasmotor.

Das Lavage-System 50 weist ein Gehäuse 52 aus Kunststoff auf, in dem der Druckgasmotor angeordnet ist. Mit dem Gehäuse 52 ist ein Pistolengriff 54 ausgeformt, mit dem das Lavage-System mit einer Hand haltbar ist. Die Flüssigkeitsauslassöffnung 18 mündet über das Lippenventil 24 in ein Rohr 56, das wiederum in einem Trichter 58 mündet. Über den Trichter 58 wird der gepulste Flüssigkeitsstrahl abgegeben.

Die Gaseinlassöffnung 22 des Druckgasmotors ist mit einer Druckgaszuleitung 60 verbunden, die wiederum mit einer Druckgasquelle (nicht gezeigt) verbunden ist. Die Flüssigkeitszuführöffnung 16 ist mit einer Flüssigkeitszuleitung 62 verbunden über die eine medizinische Spülflüssigkeit in den Druckgasmotor eingespeist wird. In der Druckgaszuleitung 60 ist ein manuell bedienbares Drehventilelement 64 angeordnet, das über einen Abzug 66 mit der gleichen Hand bedienbar ist, mit der das Lavage-System 50 am Pistolengriff 54 gehalten wird. Mit Hilfe einer Druckfeder 68 wird der Abzug 66 von dem Pistolengriff 54 weg gedreht und damit das Drehventilelement 64 in die geschlossene Position gedreht.

An der Gasauslassöffnung 20 ist eine Gasableitung 70 angeordnet, die seitlich nach außen führt und die Gasauslassöffnung 20 mit der Umgebung des Lavage-Systems 50 verbindet. In der Mündung zur Umgebung oder in der Gasableitung 70 ist ein Filter 72 beziehungsweise ein Sterilfilter 72 angeordnet. Damit wird ein Eindringen störender Partikel in den Druckgasmotor durch die Gasableitung 70 und die Gasauslassöffnung 20 verhindert und bevorzugt auch eine Kontamination des OP-Felds verhindert.

Ein Druckgas wird über die Druckgaszuleitung 60 zu dem Ventilelement 64 geleitet. Das Ventilelement 64 kann mit dem Abzug 66 nach Art einer Pistole manuell mit der Hand bedient werden. Die Gaszuleitung 60 setzt sich hinter dem manuell bedienbaren Ventilelement 64 fort und ist an die Gasauslassöffnung 26 des Druckgasmotors angeschlossen. Dadurch kann der Druckgasmotor beziehungsweise das Lavage-System 50 mit dem Abzug 66 gesteuert werden.

In der Spülflüssigkeitszuleitung 62 kann alternativ zu dem Lippenventil 26 auch ein Rückschlagventil (nicht gezeigt) angeordnet sein. Die Druckgaszuleitung 60 und die Flüssigkeitszuleitung 62 sind bodenseitig in den Pistolengriff 54 hineingeführt.

Bevorzugt ist bei solchen Lavage-Systemen 50 eine Absaugeinrichtung (nicht gezeigt) vorgesehen, mit der überschüssige Flüssigkeit und mit der Flüssigkeit abgetragene Teile über einen separaten Trichter und ein separates Rohr (nicht gezeigt) abgesaugt und abgeleitet werden. Dazu ist vorzugsweise auch eine an der Absaugeinrichtung angeschlossene Absaugleitung bodenseitig durch den Pistolengriff 54 durchgeführt. Das Absaugrohr kann das Rohr 56 konzentrisch umgeben.

Wenn das Ventilelement 64 mit dem Abzug 66 bedient wird, wird die Druckgaszuleitung 60 durchgehend und Druckgas wird in den Druckgasmotor beziehungsweise in den hinteren Bereich 7 des Innenraums eingespeist. Durch die so entstehende Druckdifferenz zwischen der Vorderseite (in Figur 6 links) des Vibrationskörpers 10 und der Rückseite (in Figur 6 rechts) des Vibrationskörpers 10 wir der Vibrationskörper 10 in Bewegung gesetzt und der Druckgasmotor arbeitet, wie zuvor zu den Figuren 1 bis 4 beschrieben. Gleichzeitig strömt die Spülflüssigkeit, die durch die Flüssigkeitszuleitung 62 aus einem externen Flüssigkeitsresevoir (nicht gezeigt) gefördert wird, periodisch in den Zwischenraum 8 zwischen dem Kolben 12 und der vorderen Wandung 5 des Druckgasmotors.

Die Spülflüssigkeit wird aus dem Druckgasmotor mit periodischen Stößen durch das Lippenventil 24 und das Rohr 56 und die Flüssigkeitsauslassöffnung 18 ausgestoßen, solange das Druckgas an der Gaseinlassöffnung 22 anliegt und die Druckluft oder das Druckgas durch die Gaseinlassöffnung 22 in den Druckgasmotor hinein und durch diesen hindurch strömt. Der Prozess wird beendet, wenn das Drehventilelement 64 nicht mehr bedient wird und dadurch die Gaszuleitung 60 unterbrochen beziehungsweise geschlossen wird. Die Rückstellung des Drehventilelements 64 erfolgt mit Hilfe der elastischen Feder 68. Durch das Federelement 14 im Druckgasmotor wird der Kolben 12 und durch die Gasfederung auf der Vorderseite des Vibrationskörpers 10 im hinteren Bereich des Innenraums 7 werden der Kolben 12 und der Vibrationskörper 10 dann in die gezeigte Ausgangsstellung gebracht und das Lavage-System 50 ist sofort wieder einsatzbereit.

Anstatt nur einen Druckgasmotor 1 kann ein Lavage-System 50 auch zwei oder mehr Druckgasmotoren 1 aufweisen, deren Druckgasanschlüsse parallel geschaltet sind. Hierdurch wird eine Verstärkung des erzeugten Sprühstrahls bewirkt oder es wird ein höher gepulster Ausstoß erreicht, beispielsweise ein Spülflüssigkeitsstrahl mit doppelter Frequenz erreicht.

Bei allen Ausführungsformen der Druckgasmotoren kann auch vorgesehen sein, dass ein zusätzliches Federelement (nicht gezeigt), wie beispielsweise eine weitere Druckfeder, zur Unterstützung oder als Alternative zur Gasfederung im hinteren Bereich 7 des Innenraums zwischen dem Vibrationskörper und der Übergangswandung 4 vorgesehen ist. Mit diesem zusätzlichen Federelement wird der Vibrationskörper 10 in Richtung Rückseite 6 des Innenraums gedrückt. Ferner kann dieses zweite Federelement auch zum Einstellen der Arbeitsfrequenz des Vibrationskörpers 10 und damit zur Feinabstimmung des Druckgasmotors 1 genutzt werden.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Druckgasmotor
- 2: Gehäuse / Seitliche Wandung des vorderen Bereichs des Innenraums
- 3: Gehäuse / Seitliche Wandung des hinteren Bereichs des Innenraums
- 4: Gehäuse / Übergangswandung zwischen dem vorderen Bereich und dem hinteren Bereich des Innenraums
- 5: Gehäuse / Deckplatte und Vorderseite des Innenraums
- 6: Gehäuse / Rückplatte und Rückseite des Innenraums
- 7: Hinterer Bereich des Innenraums
- 8: Vorderer Bereich des Innenraums
- 10: Vibrationskörper
- 12: Kolben
- 14: Federelement / Druckfeder
- 16: Flüssigkeitseinlassöffnung
- 18: Flüssigkeitsauslassöffnung
- 20: Gasauslassöffnung
- 22: Gaseinlassöffnung
- 24: Lippenventil
- 26: Lippenventil
- 28: Kanal
- 30: Umlaufende Nut
- 32: Umlaufende Nut
- 34: Vorsprung
- 36: Kanal
- 38: Kanal
- 40: Mündung
- 42: Mündung
- 50: Lavage-System
- 52: Gehäuse
- 54: Pistolengriff
- 56: Rohr
- 58: Trichter
- 60: Druckgaszuleitung
- 62: Flüssigkeitszuleitung
- 64: Ventilelement / Drehventilelement
- 66: Abzug
- 68: Feder
- 70: Gasableitung
- 72: Filter

## Patentansprüche

1. Lavage-System (50) aufweisend zumindest einen Druckgasmotor (1), der Druckgasmotor (1) aufweisend einen Vibrationskörper (10), der mit einem durch den Druckgasmotor (1) geleiteten Druckgas in Schwingung versetzbar ist, und einen Kolben (12), der mit einem Rückstellelement (14) federnd gelagert ist, wobei der Vibrationskörper (10) und der Kolben (12) derart positioniert und gelagert sind, dass der Vibrationskörper (10) bei der Schwingung wiederholt auf den Kolben (12) trifft und diesen gegen das Rückstellelement (14) auslenkt, wobei der Kolben (12) von dem Rückstellelement (14) in eine Position überführbar ist, in der der Vibrationskörper (10) bei seiner Schwingung den Kolben (12) trifft, so dass der Vibrationskörper (10) bei der Schwingung wiederholt auf den Kolben (12) trifft, und wobei die Bewegung des Kolbens (12) als Antrieb des Druckgasmotors (1) nutzbar ist, wobei mit dem Druckgasmotor (1) oder den Druckgasmotoren (1) ein periodischer Sprühstoß mit einer Flüssigkeit erzeugbar ist.

2. Lavage-System (50) nach Anspruch 1, **dadurch gekennzeichnet, dass** das Rückstellelement (14) während des Betriebs des Druckgasmotors (1) zumindest zeitweise eine in Richtung des Vibrationskörpers (10) wirkende Kraft auf den Kolben (12) ausübt.

3. Lavage-System (50) nach Anspruch 1 oder 2, **dadurch gekennzeichnet, dass** der Druckgasmotor (1) einen Innenraum (7, 8) aufweist, wobei der Vibrationskörper (10) und der Kolben (12) in dem Innenraum (7, 8) linear beweglich angeordnet sind und der Innenraum (7, 8) durch eine Vorderseite (5), eine geschlossene Rückseite (6) und eine umlaufende Seitenwand (2, 3, 4) begrenzt ist, wobei der Kolben (12) zwischen der Vorderseite (5) und dem Vibrationskörper (10) angeordnet ist.

4. Lavage-System (50) nach Anspruch 3, **dadurch gekennzeichnet, dass** der Innenraum (7, 8) einen vorderen Bereich (8) mit einer kleinen Querschnittsfläche und einen hinteren Bereich (7) mit einer großen Querschnittsfläche aufweist, die größer als die kleine Querschnittsfläche ist, wobei der Kolben (12) in dem vorderen Bereich (8) des Innenraums zwischen der Vorderseite (5) und dem Vibrationskörper (10) linear beweglich angeordnet ist.

5. Lavage-System (50) nach Anspruch 4, **dadurch gekennzeichnet, dass** der Vibrationskörper (10) einen vorderen Teil mit einer kleinen Querschnitt passend zur kleinen Querschnittsfläche des vorderen Bereichs (8) des Innenraums aufweist, so dass der vordere Teil des Vibrationskörpers (10) den vorderen Bereich (8) des Innenraums an der der Rückseite (6) zugewandten Seite im Betrieb des Druckgasmotors (1) zumindest zeitweise abschließt, und der Vibrationskörper (10) einen hinteren Teil mit einem großen Querschnitt passend zur großen Querschnittsfläche des hinteren Bereichs (7) des Innenraums aufweist, so dass der hintere Teil des Vibrationskörpers (10) den hinteren Bereich (7) des Innenraums in zwei Teile trennt.

6. Lavage-System (50) nach einem der Ansprüche 3 bis 5, **dadurch gekennzeichnet, dass**
in der Seitenwand (3) im hinteren Bereich (7) des Innenraums eine Gaseinlassöffnung (22) zum Einspeisen eines Gases in den Innenraum (7, 8) vorgesehen ist und in der Seitenwand (2) im vorderen Bereich (8) des Innenraums eine Gasauslassöffnung (20) zum Abführen des Gases aus dem Innenraum (7, 8) vorgesehen ist.

7. Lavage-System (50) nach Anspruch 6, **dadurch gekennzeichnet, dass** der Innenraum (7, 8) zwischen dem Kolben (12) und der Rückseite (6) des Innenraums (7, 8) bis auf die Gaseinlassöffnung (22) und die Gasauslassöffnung (20) geschlossen ist.

8. Lavage-System (50) nach Anspruch 6 oder 7, **dadurch gekennzeichnet, dass** in dem Vibrationskörper (10) wenigstens ein Kanal (28, 36, 38) zur Gasleitung vorgesehen ist, wobei der Kanal (28) oder die Kanäle (36, 38) den hinteren Bereich (7) des hinteren Innenraums (7) zwischen dem Vibrationskörper (10) und der Rückseite (6) des Innenraums (7, 8) abhängig von der Position des Vibrationskörpers (10) in dem Innenraum (7, 8) mit der Gaseinlassöffnung (22) oder mit der Gasauslassöffnung (20) verbindet oder verbinden.

9. Lavage-System (50) nach Anspruch 8, **dadurch gekennzeichnet, dass** der Vibrationskörper (10) in einer ersten Position den hinteren Bereich (7) des Innenraums zwischen dem Vibrationskörper (10) und der Rückseite (6) des Innenraums durch den wenigstens einen Kanal (28, 38) mit der Gaseinlassöffnung (22) verbindet und von der Gasauslassöffnung (20) trennt und in einer zweiten Position den hinteren Bereich (7) des Innenraums zwischen dem Vibrationskörper (10) und der Rückseite (6) des Innenraums durch den wenigstens einen Kanal (28, 36) mit der Gasauslassöffnung (20) verbindet und von der Gaseinlassöffnung (22) trennt.

10. Lavage-System (50) nach Anspruch 9, **dadurch gekennzeichnet, dass** der Vibrationskörper (10) in einer dritten Position zwischen der ersten Position und der zweiten Position sowohl die Gaseinlassöffnung (22) als auch die Gasauslassöffnung (20) abdeckt.

11. Lavage-System (50) nach einem der Ansprüche 8 bis 10, **dadurch gekennzeichnet, dass**
der zumindest eine gasdurchlässige Kanal (28, 38) sich von der der Rückseite (6) zugewandten Seite des Vibrationskörpers (10) bis zur seitlichen Mantelfläche des hinteren Teils des Vibrationskörpers (10) erstreckt und der gleiche Kanal (28) oder ein anderer der gasdurchlässigen Kanäle (36) sich von der der Rückseite (6) zugewandten Seite des Vibrationskörpers (10) bis zur seitlichen Mantelfläche des vorderen Teils des Vibrationskörpers (10) erstreckt, wobei bevorzugt der Vibrationskörper (10) nur einen als T-Stück ausgebildeten gasdurchlässigen Kanal (28) aufweist, der sich von der der Rückseite (6) zugewandten Seite des Vibrationskörpers (10) bis zur seitlichen Mantelfläche des hinteren Teils des Vibrationskörpers (10) und zur seitlichen Mantelfläche des vorderen Teils des Vibrationskörpers (10) erstreckt.

12. Lavage-System (50) nach einem der Ansprüche 3 bis 11, **dadurch gekennzeichnet, dass**
im vorderen Bereich (8) des Innenraums in der Seitenwand (2) des Innenraums (8) eine Flüssigkeitseinlassöffnung (16) vorgesehen ist und in der Vorderseite (5) des Innenraums (8) eine Flüssigkeitsauslassöffnung (18) vorgesehen ist, wobei bevorzugt an zumindest einer der Flüssigkeitsöffnungen (16, 18) ein Ventil (24, 26), insbesondere ein Lippenventil (24, 26) angeordnet ist.

13. Lavage-System (50) nach Anspruch 12, **dadurch gekennzeichnet, dass** an der Flüssigkeitseinlassöffnung (16) ein Ventil (26) angeordnet ist, das öffnet, wenn im vorderen Bereich (8) des Innenraums ein geringer Druck durch eine Bewegung des Kolbens (12) von der Vorderseite (5) weg entsteht, und an der Flüssigkeitsauslassöffnung (18) ein Ventil (24) angeordnet ist, das öffnet, wenn im vorderen Bereich (8) des Innenraums ein hoher Druck durch eine Bewegung des Kolbens (12) auf die Vorderseite (5) zu entsteht.

14. Lavage-System (50) nach Anspruch 12 oder 13, **dadurch gekennzeichnet, dass**
die Flüssigkeitseinlassöffnung (16) im Betrieb des Druckgasmotors (1) zumindest zeitweise nicht vom Kolben (12) abgedeckt ist und die Flüssigkeitseinlassöffnung (16) im nicht abgedeckten Zustand zwischen dem Kolben (12) und der Vorderseite (5) des Innenraums (8) angeordnet ist.

15. Lavage-System (50) nach einem der Ansprüche 12 bis 14, **dadurch gekennzeichnet, dass**
an der Flüssigkeitseinlassöffnung (16) ein Rohr oder eine Schlauch (62) mit einem Rückschlagventil angeschlossen ist, das sich bei einem Unterdruck im vorderen Bereich (8) des Innenraums zwischen dem Kolben (12) und der Vorderseite (5) des Innenraums (8) öffnet und eine Flüssigkeitszufuhr in den vorderen Bereich (8) des Innenraums ermöglicht.

16. Lavage-System (50) nach einem der vorangehenden Ansprüche, **dadurch gekennzeichnet, dass**
das Rückstellelement (14) eine elastische Druckfeder (14) ist, die bevorzugt im vorderen Bereich (8) des Innenraums zwischen dem Kolben (12) und der Vorderseite (5) des Innenraums (7, 8) angeordnet ist.

17. Lavage-System (50) nach einem der Ansprüche 3 bis 16, **dadurch gekennzeichnet, dass**
der Innenraum (7, 8) zumindest bereichsweise zylindrisch ist oder im Bereich eines Arbeitsraums (7) des Vibrationskörpers (10) und/oder des Kolbens (12) oder im gesamten Hubraum (7, 8) des Kolbens (12) und des Vibrationskörpers (10) zylindrisch ist.

18. Lavage-System (50) nach einem der vorangehenden Ansprüche, **dadurch gekennzeichnet, dass**
der Vibrationskörper (10) eine Dichte von mindestens 4 g/cm³ hat, bevorzugt eine Dichte von mindestens 7 g/cm³ hat.

19. Lavage-System (50) nach einem der vorangehenden Ansprüche, **dadurch gekennzeichnet, dass**
der Kolben (12) mit dem Rückstellelement (14) in eine Position gebracht wird, in der die dem Kolben (12) zugewandten Seite des Vibrationskörpers (10) bei maximaler Auslenkung des Vibrationskörpers (10) in Richtung des Kolbens (12) auf die dem Vibrationskörper (10) zugewandte Seite des Kolbens (12) trifft und diesen in Richtung von dem Vibrationskörper (10) weg beschleunigt.

20. Verfahren zum Erzeugen eines Sprühstoßes mit einem Druckgas, wobei in einer ersten Position eines Vibrationskörpers (10) in einem Innenraum (7, 8) ein hinterer Bereich (8) des Innenraums zwischen dem Vibrationskörper (10) und einer Rückseite (6) des Innenraums (7, 8) durch einen ersten Kanal (28, 38) im Vibrationskörper (10) mit einer Gaseinlassöffnung (22) verbunden ist und ein Druckgas durch eine Gaseinlassöffnung (22) und den ersten Kanal (28, 38) in den hinteren Bereich (7) des Innenraums zwischen dem Vibrationskörper (10) und der Rückseite (6) des Innenraums (7, 8) eingeleitet wird;
durch den sich zwischen dem Vibrationskörper (10) und der Rückseite (6) des Innenraums (7, 8) hinteren Bereich (7) des Innenraums höheren Druck und dem auf der gegenüberliegenden Seite des Vibrationskörper (10) lastenden niedrigeren Druck der Vibrationskörper (10) in Richtung einer Vorderseite (5) des Innenraums (7, 8) beschleunigt wird;
durch die Bewegung des Vibrationskörpers (10) in Richtung der Vorderseite (5) des Innenraums (7, 8) die Verbindung von dem hinteren Bereich (7) des Innenraums zwischen dem Vibrationskörper (10) und der Rückseite (6) des Innenraums zu der Gaseinlassöffnung (22) getrennt wird;
der Vibrationskörper (10) durch die Bewegung in Richtung der Vorderseite (5) des Innenraums (7, 8) mit einem vorderen Teil des Vibrationskörpers (10) in einem vorderen Bereich (8) des Innenraums (7, 8) auf einen Kolben (12) trifft und diesen in Richtung der Vorderseite (5) des Innenraums (7, 8) beschleunigt wird, wobei ein Rückstellelement (14) durch die Auslenkung des Kolbens (12) Energie aufnimmt und speichert;
durch die Bewegung des Vibrationskörpers (10) in Richtung der Vorderseite (5) des Innenraums (7, 8) der hintere Bereich (7) des Innenraums zwischen dem Vibrationskörper (10) und der Rückseite (6) des Innenraums durch den ersten Kanal (28) oder einen zweiten Kanal (36) im Vibrationskörper (10) mit einer Gasauslassöffnung (20) verbunden wird;
das Gas aus dem hinteren Bereich (7) des Innenraums zwischen dem Vibrationskörper (10) und der Rückseite (6) des Innenraums (7, 8) durch den ersten Kanal (28) oder den zweiten Kanal (36) und durch die Gasauslassöffnung (20) ausströmt, bevorzugt an die Umgebung abgegeben wird;
der Kolben (12) durch die Abgabe der Energie des Rückstellelements (14) in Richtung der Rückseite (6) des Innenraums (7, 8) beschleunigt wird;
der Vibrationskörper (10) durch das Auftreffen des Kolbens (12) und/oder durch eine Gasfeder und/oder ein zweites Rückstellelement in Richtung der Rückseite (6) des Innenraums (7, 8) beschleunigt wird;
durch die umgekehrte Bewegung des Vibrationskörpers (10) in Richtung der Rückseite (6) des Innenraums (7, 8) die Verbindung von dem hinteren Bereich (7) des Innenraums zwischen dem Vibrationskörper (10) und der Rückseite (6) des Innenraums (7, 8) zu der Gasauslassöffnung (20) getrennt wird; und durch die umgekehrte Bewegung des Vibrationskörpers (10) in Richtung der Rückseite (6) des Innenraums (7, 8) der hintere Bereich (7) des Innenraums zwischen dem Vibrationskörper (10) und der Rückseite (6) des Innenraums (7, 8) durch den ersten Kanal (28, 38) mit der Gaseinlassöffnung (22) verbunden wird und der Vibrationskörper (10) in die erste Position überführt wird, wobei
bei einer Bewegung des Kolbens (12) von der Rückseite (6) des Innenraums (7, 8) weg eine Spülflüssigkeit oder ein Flüssigkeit-Gas-Gemisch aus dem vorderen Bereich (8) des Innenraums zwischen dem Kolben (12) und der Vorderseite (5) des Innenraums (8) durch eine Flüssigkeitsauslassöffnung (18) an der Vorderseite (5) des Innenraums (7, 8) hinaus gepresst wird und
bei einer Bewegung des Kolbens (12) auf die Rückseite (6) des Innenraums (7, 8) zu eine Flüssigkeit oder ein Flüssigkeit-Gas-Gemisch in den vorderen Bereich (8) des Innenraums zwischen dem Kolben (12) und der Vorderseite (5) des Innenraums (8) durch eine Flüssigkeitseinlassöffnung (16) hinein gedrückt oder gezogen wird.

21. Verfahren nach Anspruch 20, **dadurch gekennzeichnet, dass**
der der Vorderseite (5) des Innenraums (7, 8) zugewandte Teil des hinteren Bereichs (7) des Innenraums, der auf der der Rückseite (6) zugewandten Seite dieses Teils des Innenraums (7) durch den hinteren Teil des Vibrationskörpers (10) verschlossen ist, als Gasfeder zum Beschleunigen des Vibrationskörpers (10) in Richtung der Rückseite (6) des Innenraums verwendet wird.

22. Verfahren nach einem der Ansprüche 20 oder 21, **dadurch gekennzeichnet, dass**
das Verfahren unter Verwendung eines Druckgasmotors (1) eines Lavage-Systems (50) nach einem der Ansprüche 1 bis 19 durchgeführt wird.

23. Verfahren nach einem der Ansprüche 20 bis 22, **dadurch gekennzeichnet, dass**
bei der Bewegung des Kolbens (12) auf die Vorderseite (5) des Innenraums (7, 8) zu durch den Druck in dem vorderen Bereich (8) des Innenraums zwischen dem Kolben (12) und der Vorderseite (5) des Innenraums (7, 8) ein Ventil (24) an der Flüssigkeitsauslassöffnung (18) geöffnet wird und/oder offen gehalten wird und ein an die Flüssigkeitseinlassöffnung (16) angeschlossenes Ventil (26) geschlossen wird und/oder geschlossen gehalten wird und
bei der Bewegung des Kolbens (12) auf die Rückseite (6) des Innenraums (7, 8) zu durch den geringeren Druck in dem vorderen Bereich (8) des Innenraums zwischen dem Kolben (12) und der Vorderseite (5) des Innenraums (7, 8) das Ventil (24) an der Flüssigkeitsauslassöffnung (18) geschlossen wird und/oder geschlossen gehalten wird und das an die Flüssigkeitseinlassöffnung (16) angeschlossene Ventil (26) geöffnet wird und/oder offen gehalten wird.

## Claims

1. Lavage system (50) comprising at least one compressed gas motor (1), the compressed gas motor (1) comprising a vibration body (10) that can be made to vibrate by a compressed gas being conducted through the compressed gas motor (1), and a piston (12) that is supported in spring-like fashion by a restoring element (14), whereby the vibration body (10) and the piston (12) are positioned and supported appropriately such that the vibration body (10) repeatedly hits onto the piston (12) during the vibration and deflects same against the restoring element (14), whereby the piston (12) can be transitioned by the restoring element (14) into a position, in which the vibration body (10), during its vibration, hits the piston (12) such that the vibration body (10) repeatedly hits onto the piston (12) during the vibration, and whereby the motion of the plunger (12) can be used as drive of the compressed gas motor (1), whereby a periodic spray puff can be generated with a liquid by the compressed gas motor (1) or the compressed gas motors (1).

2. Lavage system (50) according to claim 1, **characterised in that**,
during the operation of the compressed gas motor (1), the restoring element (14) exerts, at least temporarily, a force onto the plunger (12) that acts in the direction of the vibration body (10).

3. Lavage system (50) according to claim 1 or 2, **characterised in that**
the compressed gas motor (1) comprises an internal space (7, 8), whereby the vibration body (10) and the piston (12) are arranged such as to be linearly mobile in the internal space (7, 8), and the internal space (7, 8) is bounded by a front side (5), a closed rear side (6), and a circumferential side wall (2, 3, 4), whereby the piston (12) is arranged between the front side (5) and the vibration body (10).

4. Lavage system (50) according to claim 3, **characterised in that**
the internal space (7, 8) comprises a front area (8) with a small cross-sectional area and a rear area (7) with a large cross-sectional area that is larger than the small cross-sectional area, whereby the piston (12) is arranged in the front area (8) of the internal space, between the front side (5) and the vibration body (10), such as to be linearly mobile.

5. Lavage system (50) according to claim 4, **characterised in that**
the vibration body (10) comprises a front part with a small cross-section that matches the small cross-sectional area of the front area (8) of the internal space such that the front part of the vibration body (10) closes, at least temporarily, the front area (8) of the internal space on the side facing the rear side (6) during the operation of the compressed gas motor (1), and the vibration body (10) comprises a rear part with a large cross-section that matches the large cross-sectional area of the rear area (7) of the internal space such that the rear part of the vibration body (10) separates the rear area (7) of the internal space into two parts.

6. Lavage system (50) according to any one of the claims 3 to 5, **characterised in that**
a gas inlet opening (22) for feeding a gas into the internal space (7, 8) is provided in the side wall (3) in the rear area (7) of the internal space, and a gas outlet opening (20) for discharge of the gas from the internal space (7, 8) is provided in the side wall (2) in the front area (8) of the internal space.

7. Lavage system (50) according to claim 6, **characterised in that**
the internal space (7, 8) is closed between the plunger (12) and the rear side (6) of the internal space (7, 8) except for the gas inlet opening (22) and the gas outlet opening (20).

8. Lavage system (50) according to claim 6 or 7, **characterised in that**
at least one channel (28, 36, 38) for gas conduction is provided in the vibration body (10), whereby the channel (28) connects or the channels (36, 38) connect the rear area (7) of the rear internal space (7) between the vibration body (10) and the rear side (6) of the internal space (7, 8) to the gas inlet opening (22) or to the gas outlet opening (20) depending on the position of the vibration body (10) in the internal space (7, 8).

9. Lavage system (50) according to claim 8, **characterised in that**
the vibration body (10), being in a first position, connects the rear area (7) of the internal space between the vibration body (10) and the rear side (6) of the internal space to the gas inlet opening (22) through the at least one channel (28, 38) and separates it from the gas outlet opening (20), and, being in a second position, connects the rear area (7) of the internal space between the vibration body (10) and the rear side (6) of the internal space to the gas outlet opening (20) through the at least one channel (28, 36) and separates it from the gas inlet opening (22).

10. Lavage system (50) according to claim 9, **characterised in that**
the vibration body (10), being in a third position between the first position and the second position, covers both the gas inlet opening (22) and the gas outlet opening (20).

11. Lavage system (50) according to any one of the claims 8 to 10, **characterised in that**
the at least one gas-permeable channel (28, 38) extends from the side of the vibration body (10) facing the rear side (6) to the lateral jacket surface of the rear part of the vibration body (10), and the same channel (28) or any other of the gas-permeable channels (36) extends from the side of the vibration body (10) facing the rear side (6) to the lateral jacket surface of the front part of the vibration body (10), whereby the vibration body (10) preferably comprises only one gas-permeable channel (28) designed as a T-piece that extends from the side of the vibration body (10) facing the rear side (6) to the lateral jacket surface of the rear part of the vibration body (10) and to the lateral jacket surface of the front part of the vibration body (10).

12. Lavage system (50) according to any one of the claims 3 to 11, **characterised in that**
a liquid inlet opening (16) is provided in the front area (8) of the internal space, in the side wall (2) of the internal space (8), and a liquid outlet opening (18) is provided in the front side (5) of the internal space (8), whereby a valve (24, 26), in particular a lip valve (24, 26), is preferably provided on at least one of the liquid openings (16, 18).

13. Lavage system (15) according to claim 12, **characterised in that**
a valve (26) is arranged on the liquid inlet opening (16) and opens when a low pressure is generated in the front area (8) of the internal space through a motion of the plunger (12) away from the front side (5), and **in that** a valve (24) is arranged on the liquid outlet opening (18) that opens when a high pressure is generated in the front area (8) of the internal space through a motion of the plunger (12) toward the front side (5).

14. Lavage system (50) according to claim 12 or 13, **characterised in that**
the liquid inlet opening (16) is not covered by the plunger (12), at least temporarily, during operation of the compressed gas motor (1), and **in that** the liquid inlet opening (16), being in the non-covered state, is arranged between the plunger (12) and the front side (5) of the internal space (8).

15. Lavage system (50) according to any one of the claims 12 to 14, **characterised in that**
a tube or a hose (62) with a non-return valve, which opens in the presence of a negative pressure in the front area (8) of the internal space between the plunger (12) and the front side (5) of the internal space (8) and enables a supply of liquid into the front area (8) of the internal space, is connected to the liquid inlet opening (16).

16. Lavage system (50) according to any one of the preceding claims, **characterised in that**
the restoring element (14) is an elastic compression spring (14), which preferably is arranged in the front area (8) of the internal space between the plunger (12) and the front side (5) of the internal space (7, 8).

17. Lavage system (50) according to any one of the claims 3 to 16, **characterised in that**
the internal space (7, 8) is cylindrical, at least in areas thereof, or is cylindrical in the area of a working space (7) of the vibration body (10) and/or of the plunger (12) or in the entire swept volume (7, 8) of the plunger (12) and the vibration body (10).

18. Lavage system (50) according to any one of the preceding claims, **characterised in that**
the vibration body 10) has a density of at least 4 g/cm³, preferably a density of at least 7 g/cm³.

19. Lavage system (50) according to any one of the preceding claims, **characterised in that**
the piston (12) is moved by the restoring element (14) into a position, in which, upon maximal deflection of the vibration body (10) in the direction of the plunger (12), the side of the vibration body (10) facing the plunger (12) hits onto the side of the plunger (12) facing the vibration body (10) and propels same in the direction away from the vibration body (10).

20. Method for producing a spray puff with a compressed gas, whereby,
in a first position of a vibration body (10) in an internal space (7, 8), a rear area (8) of the internal space between the vibration body (10) and a rear side (6) of the internal space (7, 8) is connected to a gas inlet opening (22) through a first channel (28, 38) in the vibration body (10), and a compressed gas is being introduced through a gas inlet opening (22) and the first channel (28, 38) into the rear area (7) of the internal space between the vibration body (10) and the rear side (6) of the internal space (7, 8);
the vibration body (10) is being propelled in the direction of a front side (5) of the internal space (7, 8) by the higher pressure in the rear area (7) of the internal space between the vibration body (10) and the rear side (6) of the internal space (7, 8) and the lower pressure that acts on the opposite side of the vibration body (10);
the connection from the rear area (7) of the internal space between the vibration body (10) and the rear side (6) of the internal space to the gas inlet opening (22) is being separated by the motion of the vibration body (10) in the direction of the front side (5) of the internal space (7, 8);
the vibration body (10), due to the motion in the direction of the front side (5) of the internal space (7, 8), hits, by a front part of the vibration body (10), onto a piston (12) in a front area (8) of the internal space (7, 8) and propels the piston (12) in the direction of the front side (5) of the internal space (7, 8), whereby a restoring element (14) takes up and stores energy due to the deflection of the plunger (12);
the rear area (7) of the internal space between the vibration body (10) and the rear side (6) of the internal space is being connected to a gas outlet opening (20), due to the motion of the vibration body (10) in the direction of the front side (5) of the internal space (7, 8), through the first channel (28) or a second channel (36) in the vibration body (10);
the gas flows from the rear area (7) of the internal space between the vibration body (10) and the rear side (6) of the internal space (7, 8) through the first channel (28) or the second channel (36) and through the gas outlet opening (20), preferably is being discharged into the surroundings;
the plunger (12) is being accelerated in the direction of the rear side (6) of the internal space (7, 8) by the release of the energy of the restoring element (14); the vibration body (10) is being accelerated in the direction of the rear side (6) of the internal space (7, 8) by being hit by the plunger (12) and/or by a gas spring and/or a second restoring element;
the connection from the rear area (7) of the internal space between the vibration body (10) and the rear side (6) of the internal space (7, 8) to the gas outlet opening (20) is being separated by the reverse motion of the vibration body (10) in the direction of the rear side (6) of the internal space (7, 8); and, the rear area (7) of the internal space between the vibration body (10) and the rear side (6) of the internal space (7, 8) is being connected to the gas inlet opening (22) through the first channel (28, 38) by the reverse motion of the vibration body (10) in the direction of the rear side (6) of the internal space (7, 8), and the vibration body (10) is being transitioned into the first position, whereby,
upon a motion of the plunger (12) away from the rear side (6) of the internal space (7, 8), a rinsing liquid or a liquid-gas mixture is extruded from the front area (8) of the internal space between the plunger (12) and the front side (5) of the internal space (8) through a liquid outlet opening (18) on the front side (5) of the internal space (7, 8), and,
upon a motion of the plunger (12) towards the rear side (6) of the internal space (7, 8), a liquid or a liquid-gas mixture is being pushed or drawn into the front area (8) of the internal space between the plunger (12) and the front side (5) of the internal space (8) through a liquid inlet opening (16).

21. Method according to claim 20, **characterised in that**
the part of the rear area (7) of the internal space that faces the front side (5) of the internal space (7, 8) and is closed, on the side of this part of the internal space (7) that faces the rear side (6), by the rear part of the vibration body (10), is used as gas spring for propulsion of the vibration body (10) in the direction of the rear side (6) of the internal space.

22. Method according to any one of the claims 20 or 21, **characterised in that**
the method is carried out through the use of a compressed gas motor (1) of a lavage system (50) according to any one of the claims 1 to 19.

23. Method according to any one of the claims 20 to 22, **characterised in that**
the pressure in the front area (8) of the internal space between the plunger (12) and the front side (5) of the internal space (7, 8) opens and/or keeps open a valve (24) on the liquid outlet opening (18) during the motion of the plunger (12) towards the front side (5) of the internal space (7, 8), and closes and/or keeps closed a valve (26) connected to the liquid inlet opening (16), and the lower pressure in the front area (8) of the internal space between the plunger (12) and the front side (5) of the internal space (7, 8) closes and/or keeps close the valve (24) on the liquid outlet opening (18) during the motion of the plunger (12) towards the rear side (6) of the internal space (7, 8) and/or opens and/or keeps open the valve (26) that is connected to the liquid inlet opening (16).

## Revendications

1. Système de lavage (50) présentant au moins un moteur à gaz comprimé (1), le moteur à gaz comprimé (1) présentant un corps de vibration (10) qui peut être amené à osciller avec un gaz comprimé conduit à travers le moteur à gaz comprimé (1), et un piston (12) qui est logé de manière élastique avec un élément de rappel (14), dans lequel le corps de vibration (10) et le piston (12) sont positionnés et logés de telle sorte que le corps de vibration (10) frappe sur le piston (12) de manière répétée lors de l'oscillation et dévie celui-ci contre l'élément de rappel (14), dans lequel le piston (12) peut être transféré par l'élément de rappel (14) dans une position dans laquelle le corps de vibration (10) frappe le piston (12) lors de son oscillation de sorte que le corps de vibration (10) frappe sur le piston (12) de manière répétée lors de l'oscillation, et dans lequel le mouvement du piston (12) peut être exploité en tant qu'entraînement du moteur à gaz comprimé (1), dans lequel un jet pulvérisé périodique avec un liquide peut être généré avec le moteur à gaz comprimé (1) ou les moteurs à gaz comprimé (1).

2. Système de lavage (50) selon la revendication 1, **caractérisé en ce que** l'élément de rappel (14) exerce une force sur le piston (12) agissant en direction du corps de vibration (10) au moins par intermittence pendant le fonctionnement du moteur à gaz comprimé (1).

3. Système de lavage (50) selon la revendication 1 ou 2, **caractérisé en ce que** le moteur à gaz comprimé (1) présente un espace interne (7, 8), dans lequel le corps de vibration (10) et le piston (12) sont disposés de manière linéairement mobile dans l'espace interne (7, 8), et l'espace interne (7, 8) est limité par un côté avant (5), un côté arrière fermé (6) et une paroi latérale périphérique (2, 3, 4), dans lequel le piston (12) est disposé entre le côté avant (5) et le corps de vibration (10).

4. Système de lavage (50) selon la revendication 3, **caractérisé en ce que** l'espace interne (7, 8) présente une région avant (8) avec une petite superficie de la section et une région arrière (7) avec une grande superficie de la section qui est supérieure à la petite superficie de la section, dans lequel le piston (12) est disposé de manière linéairement mobile dans la région avant (8) de l'espace interne entre le côté avant (5) et le corps de vibration (10).

5. Système de lavage (50) selon la revendication 4, **caractérisé en ce que** le corps de vibration (10) présente une partie avant avec une petite section transversale adaptée à la petite superficie de la section de la région avant (8) de l'espace interne de sorte que la partie avant du corps de vibration (10) termine au moins par intermittence la région avant (8) de l'espace interne sur le côté tourné vers le côté arrière (6) en fonctionnement du moteur à gaz comprimé (1), et le corps de vibration (10) présente une partie arrière avec une grande section transversale adaptée à la grande superficie de la section de la région arrière (7) de l'espace interne de sorte que la partie arrière du corps de vibration (10) sépare en deux parties la région arrière (7) de l'espace interne.

6. Système de lavage (50) selon une des revendications 3 à 5, **caractérisé en ce que**
une ouverture d'entrée gazeuse (22) pour l'alimentation d'un gaz dans l'espace interne (7, 8) est prévue dans la paroi latérale (3) dans la région arrière (7) de l'espace interne et une ouverture de sortie gazeuse (20) pour l'évacuation du gaz de l'espace interne (7, 8) est prévue dans la paroi latérale (2) dans la région avant (8) de l'espace interne.

7. Système de lavage (50) selon la revendication 6, **caractérisé en ce que** l'espace interne (7, 8) est fermé entre le piston (12) et le côté arrière (6) de l'espace interne (7, 8) hormis l'ouverture d'entrée gazeuse (22) et l'ouverture de sortie gazeuse (20).

8. Système de lavage (50) selon la revendication 6 ou 7, **caractérisé en ce que** au moins un canal (28, 36, 38) pour la conduite de gaz est prévu dans le corps de vibration (10), dans lequel le canal (28) ou les canaux (36, 38) connecte ou connectent la région arrière (7) de l'espace interne arrière (7) entre le corps de vibration (10) et le côté arrière (6) de l'espace interne (7, 8) en fonction de la position du corps de vibration (10) dans l'espace interne (7, 8) à l'ouverture d'entrée gazeuse (22) ou à l'ouverture de sortie gazeuse (20).

9. Système de lavage (50) selon la revendication 8, **caractérisé en ce que** le corps de vibration (10) connecte dans une première position la région arrière (7) de l'espace interne entre le corps de vibration (10) et le côté arrière (6) de l'espace interne à travers l'au moins un canal (28, 38) à l'ouverture d'entrée gazeuse (22) et la sépare de l'ouverture de sortie gazeuse (20), et connecte dans une deuxième position la région arrière (7) de l'espace interne entre le corps de vibration (10) et le côté arrière (6) de l'espace interne à travers l'au moins un canal (28, 36) à l'ouverture de sortie gazeuse (20) et la sépare de l'ouverture d'entrée gazeuse (22).

10. Système de lavage (50) selon la revendication 9, **caractérisé en ce que** le corps de vibration (10) recouvre dans une troisième position entre la première position et la deuxième position aussi bien l'ouverture d'entrée gazeuse (22) que l'ouverture de sortie gazeuse (20).

11. Système de lavage (50) selon une des revendications 8 à 10, **caractérisé en ce que**
l'au moins un canal perméable au gaz (28, 38) s'étend du côté du corps de vibration (10) tourné vers le côté arrière (6) jusqu'à la face d'enveloppe latérale de la partie arrière du corps de vibration (10) et le même canal (28) ou un autre des canaux perméables au gaz (36) s'étend du côté du corps de vibration (10) tourné vers le côté arrière (6) jusqu'à la face d'enveloppe latérale de la partie avant du corps de vibration (10), dans lequel le corps de vibration (10) ne présente de préférence qu'un canal perméable au gaz (28) réalisé en tant que pièce en T qui s'étend du côté du corps de vibration (10) tourné vers le côté arrière (6) jusqu'à la face d'enveloppe latérale de la partie arrière du corps de vibration (10) et jusqu'à la face d'enveloppe latérale de la partie avant du corps de vibration (10).

12. Système de lavage (50) selon une des revendications 3 à 11, **caractérisé en ce que**
une ouverture d'entrée de liquide (16) est prévue dans la région avant (8) de l'espace interne dans la paroi latérale (2) de l'espace interne (8) et une ouverture de sortie de liquide (18) est prévue dans le côté avant (5) de l'espace interne (8), dans lequel une soupape (24, 26), notamment une soupape à bec (24, 26), est de préférence disposée sur au moins une des ouvertures de liquide (16, 18).

13. Système de lavage (50) selon la revendication 12, **caractérisé en ce que** une soupape (26) qui s'ouvre lorsqu'une faible pression survient dans la région avant (8) de l'espace interne par un mouvement du piston (12) à l'écart du côté avant (5) est disposée sur l'ouverture d'entrée de liquide (16), et une soupape (24) qui s'ouvre lorsqu'une pression élevée survient dans la région avant (8) de l'espace interne par un mouvement du piston (12) vers le côté avant (5) est disposée sur l'ouverture de sortie de liquide (18).

14. Système de lavage (50) selon la revendication 12 ou 13, **caractérisé en ce que** l'ouverture d'entrée de liquide (16) n'est pas recouverte par le piston (12) au moins par intermittence en fonctionnement du moteur à gaz comprimé (1) et l'ouverture d'entrée de liquide (16) est disposée dans l'état non recouvert entre le piston (12) et le côté avant (5) de l'espace interne (8).

15. Système de lavage (50) selon une des revendications 12 à 14, **caractérisé en ce que**
un tube ou un tuyau (62) avec une soupape de non-retour qui s'ouvre en cas de dépression dans la région avant (8) de l'espace interne entre le piston (12) et le côté avant (5) de l'espace interne (8) et permet une amenée de liquide dans la région avant (8) de l'espace interne est raccordé à l'ouverture d'entrée de liquide (16).

16. Système de lavage (50) selon une des revendications précédentes, **caractérisé en ce que**
l'élément de rappel (14) est un ressort de compression élastique (14) qui est de préférence disposé dans la région avant (8) de l'espace interne entre le piston (12) et le côté avant (5) de l'espace interne (7, 8).

17. Système de lavage (50) selon une des revendications 3 à 16, **caractérisé en ce que**
l'espace interne (7, 8) est cylindrique au moins par région ou est cylindrique dans la région d'un espace de travail (7) du corps de vibration (10) et/ou du piston (12) ou dans la cylindrée (7, 8) du piston (12) et du corps de vibration (10).

18. Système de lavage (50) selon une des revendications précédentes, **caractérisé en ce que**
le corps de vibration (10) a une densité d'au moins 4 g/cm³, de préférence une densité d'au moins 7 g/cm³.

19. Système de lavage (50) selon une des revendications précédentes, **caractérisé en ce que**
le piston (12) est amené avec l'élément de rappel (14) dans une position dans laquelle le côté du corps de vibration (10) tourné vers le piston (12) frappe sur le côté du piston (12) tourné vers le corps de vibration (10) en cas de déviation maximale du corps de vibration (10) en direction du piston (12) et accélère celui-ci dans une direction à l'écart du corps de vibration (10).

20. Procédé de génération d'un jet pulvérisé avec un gaz comprimé, dans lequel une région arrière (8) d'un espace interne (7, 8) est connectée dans une première position d'un corps de vibration (10) dans l'espace interne entre le corps de vibration (10) et un côté arrière (6) de l'espace interne (7, 8) par un premier canal (28, 38) dans le corps de vibration (10) à une ouverture d'entrée gazeuse (22) et un gaz comprimé est introduit à travers une ouverture d'entrée gazeuse (22) et le premier canal (28, 38) dans la région arrière (7) de l'espace interne entre le corps de vibration (10) et le côté arrière (6) de l'espace interne (7, 8) ;
le corps de vibration (10) est accéléré en direction d'un côté avant (5) de l'espace interne (7, 8) par la pression supérieure la région arrière (7) de l'espace interne entre le corps de vibration (10) et le côté arrière (6) de l'espace interne (7, 8) et la pression inférieure pesant sur le côté opposé du corps de vibration (10) ;
la connexion de la région arrière (7) de l'espace interne entre le corps de vibration (10) et le côté arrière (6) de l'espace interne à l'ouverture d'entrée gazeuse (22) est séparée par le mouvement du corps de vibration (10) en direction du côté avant (5) de l'espace interne (7, 8) ;
le corps de vibration (10) frappe sur un piston (12) par le mouvement en direction du côté avant (5) de l'espace interne (7, 8) avec une partie avant du corps de vibration (10) dans une région avant (8) de l'espace interne (7, 8) et accélère celui-ci en direction du côté avant (5) de l'espace interne (7, 8), dans lequel un élément de rappel (14) reçoit et stocke de l'énergie par la déviation du piston (12) ;
la région arrière (7) de l'espace interne entre le corps de vibration (10) et le côté arrière (6) de l'espace interne est connectée à travers le premier canal (28) ou un second canal (36) dans le corps de vibration (10) à une ouverture de sortie gazeuse (20) par le mouvement du corps de vibration (10) en direction du côté avant (5) de l'espace interne (7, 8) ;
le gaz s'échappe de la région arrière (7) de l'espace interne entre le corps de vibration (10) et le côté arrière (6) de l'espace interne (7, 8) à travers le premier canal (28) ou le second canal (36) et à travers l'ouverture de sortie gazeuse (20), est de préférence dégagé vers l'environnement ;
le piston (12) est accéléré par le dégagement de l'énergie de l'élément de rappel (14) en direction du côté arrière (6) de l'espace interne (7, 8) ;
le corps de vibration (10) est accéléré par la frappe du piston (12) et/ou par un ressort pneumatique et/ou un second élément de rappel en direction du côté arrière (6) de l'espace interne (7, 8) ;
la connexion de la région arrière (7) de l'espace interne entre le corps de vibration (10) et le côté arrière (6) de l'espace interne (7, 8) à l'ouverture de sortie gazeuse (20) est séparée par le mouvement inverse du corps de vibration (10) en direction du côté arrière (6) de l'espace interne (7, 8) ; et
la région arrière (7) de l'espace interne entre le corps de vibration (10) et le côté arrière (6) de l'espace interne (7, 8) est connectée à travers le premier canal (28, 38) à l'ouverture d'entrée gazeuse (22) par le mouvement inverse du corps de vibration (10) en direction du côté arrière (6) de l'espace interne (7, 8) et le corps de vibration (10) est transféré dans la première position, dans lequel
un liquide de rinçage ou un mélange liquide-gaz est pressé de la région avant (8) de l'espace interne entre le piston (12) et le côté avant (5) de l'espace interne (8) à travers une ouverture de sortie de liquide (18) au-delà du côté avant (5) de l'espace interne (7, 8) lors d'un mouvement du piston (12) à l'écart du côté arrière (6) de l'espace interne (7, 8), et
un liquide ou un mélange liquide-gaz est poussé ou tiré dans la région avant (8) de l'espace interne entre le piston (12) et le côté avant (5) de l'espace interne (8) à travers une ouverture d'entrée de liquide (16) lors d'un mouvement du piston (12) vers le côté arrière (6) de l'espace interne (7, 8).

21. Procédé selon la revendication 20, **caractérisé en ce que**
la partie de la région arrière (7) de l'espace interne tournée vers le côté avant (5) de l'espace interne (7, 8) qui est fermée sur le côté tourné vers le côté arrière (6) de cette partie de l'espace interne (7) par la partie arrière du corps de vibration (10) est utilisée en tant que ressort pneumatique pour l'accélération du corps de vibration (10) en direction du côté arrière (6) de l'espace interne.

22. Procédé selon une des revendications 20 ou 21, **caractérisé en ce que**
le procédé est réalisé en utilisant un moteur à gaz comprimé (1) d'un système de lavage (50) selon une des revendications 1 à 19.

23. Procédé selon une des revendications 20 à 22, **caractérisé en ce que**
une soupape (24) est ouverte et/ou est maintenue ouverte au niveau de l'ouverture de sortie de liquide (18) et une soupape (26) raccordée à l'ouverture d'entrée de liquide (16) est fermée et/ou est maintenue fermée lors du mouvement du piston (12) vers le côté avant (5) de l'espace interne (7, 8) par la pression dans la région avant (8) de l'espace interne entre le piston (12) et le côté avant (5) de l'espace interne (7, 8), et
la soupape (24) est fermée et/ou est maintenue fermée au niveau de l'ouverture de sortie de liquide (18) et la soupape (26) raccordée à l'ouverture d'entrée de liquide (16) est ouverte et/ou est maintenue ouverte lors du mouvement du piston (12) vers le côté arrière (6) de l'espace interne (7, 8) par la pression inférieure dans la région avant (8) de l'espace interne entre le piston (12) et le côté avant (5) de l'espace interne (7, 8).
